# EUROPEAN PATENT APPLICATION

(11) **EP 4 623 842 A1**
(43) Date of publication of application: **01.10.2025**
(21) Application number: 23822412.5
(22) Date of filing: 22.11.2023
(51) Int. Cl.: A61B 17/16

(54) **RUPTURING TOOL AND RUPTURING SYSTEM WITH BILATERALLY ARTICULATED RUPTURING ELEMENT**

(30) Priority: 23.11.2022 EP 22383124
(71) Applicant: Abanza Tecnomed, S.L., 31192 Mutilva, Navarra (ES)
(72) Inventor: ABASCAL RUBIO, José Manuel, 31192 Mutilva, Navarra (ES); ABASCAL AZANZA, Juan, 31006 Pamplona, Navarra (ES); MENAUT BELTRÁN, Ander, 20018 San Sebastián, Guipúzcoa (ES); MAYA PABOLAZA, Alejandro, 31002 Pamplona, Navarra (ES)
(74) Representative: ABG Intellectual Property Law, S.L.
(86) International application number: PCT/ES2023/070694
(87) International publication number: WO 2024/110681

(57) **Abstract**

The present invention relates to a rupturing tool for surgical interventions and to a rupturing system comprising the rupturing tool and a rupturing movement generator device. The rupturing tool and system are intended for the field of traumatology, particularly for the creation of bone tunnels suitable for the proper anatomical reconstruction of tendons and ligaments; for example, for the creation of bone tunnels in interventions for reconstructing the anterior cruciate ligament (ACL) of the knee joint and in interventions for repairing the supraspinatus tendon of the shoulder joint.

## Description

### OBJECT OF THE INVENTION

The present invention relates to a rupturing tool for surgical interventions and to a rupturing system comprising the rupturing tool and a rupturing movement generator device. The rupturing tool and system are intended for the field of traumatology, particularly for the creation of bone tunnels suitable for the proper anatomical reconstruction of tendons and ligaments; for example, for the creation of bone tunnels in interventions for reconstructing the anterior cruciate ligament (ACL) of the knee joint and in interventions for repairing the supraspinatus tendon of the shoulder joint.

### BACKGROUND OF THE INVENTION

One of the most common injuries in the field of traumatology is joint tendon and ligament ruptures.

Among the most common tendon ruptures are tendon rupture in the rotator cuff of the shoulder, mainly the supraspinatus muscle tendon. This injury represents the main cause of shoulder instability and is present in 20.7% of the general population, with a prevalence that increases with age *("*Prevalence and risk factors of a rotator cuff tear in the general population", Yamamoto A et at / J Shoulder Elbow Surg. 2010 Jan; 19(1):116-20*).*

Screws which are screwed into the head of the humerus are often used during supraspinatus tendon reconstruction surgery. These screws carry suture threads or suture bands, which are passed through the damaged end of the tendon, in order to pull on it, reposition it, and fix it on the original area of insertion.

In many cases, a problem which prevents a successful reconstruction is that the screws carrying the sutures only provide surface compression at the end of the tendon, on the original area of insertion of the tendon, an area which is furthermore reduced by the screw itself, such that the healing process is hindered. If the tendon does not heal, the surgery is inadequate because it is not only intended for repositioning the tendon in the bone, but also for promoting the tendon-bone healing process for a proper reconstruction of said damaged tendon (Castagna et al. 2018, Arthroscopic Transosseous Rotator Cuff Repair)*.*

With respect to ligaments, the rupture of knee joint cruciate ligaments, mainly the anterior cruciate ligament (ACL), which occurs in patients of all ages, stands out as the most common ruptures.

In most cases, patients suffering from an ACL rupture must undergo a surgical procedure consisting of removing the damaged ACL and replacing it with an implant, the two ends of which are implanted and fixed in respective tibial and femoral bone tunnels.

As with tendons, to achieve anatomical ACL reconstruction, a system which allows restoring the original areas of insertion must be provided. Unfortunately, with the current techniques, 70% of patients develop osteoarthritis between 15 and 20 years after surgery.

To successfully restore anatomical bone insertions of the original ACL, solutions describing arthroscopic guides which allow creating two or more contiguous bone tunnels that are subsequently attached by means of dilator are known in the current state of the art. For example, document US 2019/0192278 describes an arthroscopic guide which allows creating three contiguous bone tunnels that are subsequently attached by means of a dilator.

However, a first problem associated with multi-tunnel rupturing systems of this type is that the tibia cannot be accessed "from the inside out", so they do not allow carrying out the "all-in" cruciate ligament anatomical reconstruction techniques. A second problem is that, by accessing the lateral condyle of the femur through the medial portal, it is impossible to make a sufficiently transverse femoral bone tunnel, so the degrees of twisting with respect to the original ACL are lost. The consequences of failing to completely restore the twisting biomechanics of the original ACL are widely described in the literature: rotational instability of the knee and a medium-term degenerative arthrosis.

The solution to these problems requires creating a bone tunnel for the passage of sutures by means of minimally invasive surgery and carving a bone housing along the original anatomical area of insertion, intended for the insertion of the damaged end of the tendon and/or of the implant used for repair or reconstruction, which is not possible with the articulated rupturing tools existing today in the state of the art, such as those described in documents US W0219US22632 and US9254138.

These articulated rupturing tools of the state of the art are intended for rupturing tissue during minimally invasive vertebral discectomy surgeries. Both rupturing tools comprise a rotary movement transmission shaft and a rupturing element linked in an articulated manner to the rotary movement transmission shaft. However, the solutions described in both documents provide unilateral tools with a large number of parts, which increase manufacturing and assembly costs and do not provide the rupturing tool with the rigidity required for carving the bone tissue.

Moreover, both rupturing tools of the state of the art only allow sweeping one of the sides of the sagittal plane of the rupturing tool; the rupturing element starts from a straight position in which the rupturing element is coaxial with the rotary movement transmission shaft, to an angled position in which the rupturing element is angled with respect to said shaft but only with respect to one of the sides, not with respect to the opposite side.

In intervertebral discectomies, the tissue to be ruptured is soft tissue, so small-sized and lightweight movement generator devices, i.e., microdrills, are used, hence a simple unilateral articulation of the rupturing tool once it is introduced in the intervertebral space is sufficient since the system comprising the rupturing tool and microdrill can be handled and rotated with ease due to it being lightweight.

However, heavier drills are used in minimally invasive surgeries for the anatomical widening of bone tunnels in large bones such as the tibia, femur, and humerus, and in this application, the bilaterally articulated rupturing tool prevents the complication of having to rotate the entire system in order to carve the bone bilaterally.

Therefore, in view of these problems, there is a need to provide bilaterally articulated rupturing tools and systems with sufficient rigidity to carve bone tissue in larger bones, such that they allow creating a straight bone tunnel and then widening its intraarticular outlet opening bilaterally, in a simple, reliable, reproducible, and precise manner, providing a bone housing suitable for the anatomical reinsertion and regeneration of the end of a damaged tendon or ligament.

### DESCRIPTION OF THE INVENTION

The present invention proposes a solution to the preceding problems by means of a rupturing tool for surgical interventions according to claim 1 and rupturing systems for surgical interventions according to claims 15 and 16. The dependent claims define preferred embodiments of the invention.

In a first inventive aspect, the invention provides *a bilateral rupturing tool for minimally invasive surgical interventions comprising:*
- *a longitudinal element oriented according to a main longitudinal axis which in turn comprises:*
   ∘ *a first longitudinal body configured for performing a linear movement along the main longitudinal axis and comprising a first distal end,*
   ∘ *a second longitudinal body, arranged parallel to the first longitudinal body, configured for performing a linear movement along the main longitudinal axis and comprising a second distal end;*
- *a rupturing assembly which in turn comprises:*
   ∘ *a rupturing element comprising an axis of rupture and configured for receiving and performing a rupturing movement about the axis of rupture and for performing an angular movement in a main plane, forming a plurality of angles α with respect to the main longitudinal axis comprised in a main plane, said main plane containing the main longitudinal axis and the axis of rupture; and*
   ∘ *a movement transfer element, comprising a distal portion, wherein the movement transfer element:*
      *is arranged inside the longitudinal element, between the first longitudinal body and the second longitudinal body;*
      *is attached to the rupturing element by the distal portion, and*
      *is configured for receiving and performing a rupturing movement and for transferring the rupturing movement to the rupturing element,*
- *an articulated attachment means which comprises*
   *a first section attached to the first distal end of the first longitudinal body;*
   *a second section attached to the second distal end of the second longitudinal body; and*
   *a central section comprising a through hole with the rupturing assembly going through same, the rupturing assembly being fixed to the inside of the through hole;*
   *wherein the articulated attachment means is configured for performing an angular movement in the main plane of the rupturing tool, such that:*

- *when the first longitudinal body performs linear movement in the proximal-distal direction, the second longitudinal body performs linear movement in the distal-proximal direction and the rupturing element performs angular movement in the main plane in a first direction, and*
- *when the first longitudinal body performs linear movement in the distal-proximal direction, the second longitudinal body performs linear movement in the proximal-distal direction and the rupturing element performs angular movement in the main plane in the direction opposite the first direction.*

The bilateral rupturing tool of the invention allows creating bone tunnels suitable for the anatomical reconstruction of damaged tendons and/or ligaments. In particular, the tool allows creating bone tunnels with elongated, for example, straight, curved, or angled, intraarticular outlet openings similar to the original anatomical areas of insertion, capable of housing the tendon or ligament under reconstruction at a bone depth enough to ensure its regeneration. A restoration that favors osteointegration is thus achieved.

Throughout this document, tendons shall be understood to mean bands of connective tissue configured for attaching muscles to bones and ligaments shall be understood to mean bands of connective tissue configured for attaching bones to one another.

Throughout this document, the proximal end of an element of the tool (or of the system) shall be understood to mean the end closer to the subject using said tool. In contrast, the distal end of an element of the tool (or of the system) shall be understood to mean the end farther away from the subject using said tool. Preferably, the subject or user is a doctor, a veterinarian, or a medical or veterinary professional.

First, the tool comprises a longitudinal element. "Longitudinal" shall be understood to mean that the body is made or placed in the lengthwise direction thereof, particularly oriented according to a main longitudinal axis.

Said longitudinal element in turn comprises a first longitudinal body and a second longitudinal body. Both longitudinal bodies are arranged in parallel and perform respective linear movements along the main longitudinal axis. Preferably, the first longitudinal body and the second longitudinal body are in the form of a channel, conduit, or semi-cylindrical tube, with the hollow portions of each of said bodies facing one another.

Second, the tool comprises a rupturing assembly. Said assembly in turn comprises a rupturing element and a movement transfer element attached to one another. This attachment can be a fixed attachment, such that the assembly is a one-piece assembly, or can be done by means of an intermediate attachment element, for example by means of a bearing. Furthermore, this attachment is established between the distal portion of the movement transfer element and the most proximal end of the rupturing element.

Throughout the document, "*rupturing element"* shall be understood to mean any type of element capable of rupturing, milling, cutting, reaming, polishing, compacting, or perforating a biological tissue; particularly bone tissue.

In a particular example, the rupturing element is a mill, a rupturing mill, a bit, a blade, a scraper, a rasp, a vibrating rupturing element, or a reciprocating rupturing and/or compacting element.

In another particular example, the rupturing element is a compacting mill which causes bone densification which is important for early loading, allowing the rehabilitation time required to return to the pre-injury activity level to be shortened.

In other more particular examples, the rupturing element:
- has a frustoconical geometry, with a proximal diameter greater than the distal diameter;
- has a cylindrical geometry;
- is a mixed element, wherein the proximal segment is a compacting segment and the distal segment is a rupturing segment;
- has a distal cross-section that is circular, elliptical, trilobular, polygonal or corresponds to any other geometric figure.

In a particular embodiment, the diameter of the rupturing element can be adjusted at will by the user.

This rupturing element comprises an axis of rupture; fore xample, an axis of rotation about which the element performs a rotation or an axis of vibration about which the element performs a vibratory movement. Said element is configured for receiving and performing a rupturing movement; being understood to be a movement which can be performed by the rupturing element such that said element can rupture a human tissue, preferably bone. Examples of rupturing movement are a rotational and/or reciprocating and/or vibrating movement performed about the axis of rupture.

Throughout the document, rotary or rotational movement shall be understood to mean the movement in which the rupturing element performs a (constant or non-constant) 360-degree rotation. Vibrating or vibratory movement shall be understood to mean the movement in which the rupturing element performs a preferably periodic movement oscillating about a stable equilibrium position, such that it moves between two end positions.

Additionally, the rupturing element is configured for receiving and performing an angular movement in a main plane, such that said rupturing element gradually forms a plurality of angles α (indicated as α1, α2, etc.) with respect to the main longitudinal axis. Therefore, main plane shall be understood to mean the plane containing the main longitudinal axis and the axis of rupture, in any position of said axis of rupture.

In order for this rupturing element to be able to perform the mentioned rupturing movement, the rupturing assembly further comprises a movement transfer element. This movement transfer element is arranged inside the longitudinal element, that is, between the first longitudinal body and the second longitudinal body. Preferably, the first longitudinal body and the second longitudinal body are semi-cylindrical tubes and the movement transfer element is housed inside the hollow portion of both semi-cylindrical tubes.

Preferably, the movement transfer element is flexible. In one example, the flexibility of the movement transfer element is due to a braided tubular configuration of the element. In another example, the flexibility of the movement transfer element is due to a tubular configuration comprising cuts and/or openings in the element. In another example, the flexibility of the movement transfer element is due to said element comprising a superelastic nitinol braided core and an outer polymer layer.

Since the movement transfer element is attached to the rupturing element, either directly or by means of an intermediate attachment means, the rupturing movement received and performed by the movement transfer element with respect to its axis of rupture is transferred to the rupturing element, which will perform said rupturing movement with respect to its own axis of rupture.

Preferably, the movement transfer element is in the form of a rod or elongated cylinder.

Lastly, the tool comprises an articulated attachment means. Throughout the document, the term "articulated" shall be understood as a means or element which is attached to one or more elements such that they maintain certain freedom of movement with respect to one another; as well as an element folded on itself configuring portions which maintain certain freedom of movement with respect to one another.

The articulated attachment means is therefore formed by three sections attached to one another, forming a single part:
- a first section which is in turn attached to the first longitudinal body at the distal end thereof;
- a second section which is in turn attached to the second longitudinal body at the distal end thereof; and
- a central section arranged between the first section and the second section, comprising a through hole with the rupturing assembly going through same, and said rupturing assembly being fixed to the inside of the through hole.

Preferably, the attachment between the first section and the first longitudinal body as well as the attachment between the second section and the second longitudinal body is a mechanical attachment, for example by means of a hinge. Alternatively, this attachment or these attachments can be flexible, that is, all the elements form a one-piece assembly but their attachments maintain degrees of freedom.

The articulated attachment means is configured for performing an angular movement about the through hole in the main plane of the tool, this angular movement being the result of the linear movements of the first longitudinal body and the second longitudinal body. In that sense:
- on one hand, when the first longitudinal body performs linear movement in the proximal-distal direction, the first section of the articulated attachment means also describes a proximal-distal movement. Simultaneously, the second longitudinal body performs linear movement in the distal-proximal direction and the second section of the articulated attachment means also describes a distal-proximal movement. In this way, both sections move in opposite directions about the through hole of the central section.
- On the other hand, when the first longitudinal body performs linear movement in the distal-proximal direction, the first section describes a distal-proximal movement and, simultaneously, the second longitudinal body and the second section of the articulated attachment means perform the corresponding linear movement in the proximal-distal direction.

The purpose of this angular movement is to cause the rupturing element to perform the angular movement with respect to the main longitudinal axis in the main plane. Since the rupturing assembly goes through and is fixed to the inside of the through hole of the attachment means, the angular movement of said attachment means causes the angular movement of the rupturing element. In particular, when the first section performs a proximal-distal movement, the rupturing element moves angularly on the front surface in a first direction, for example, to the left; whereas when the second section is the one which performs said proximal-distal movement, the rupturing element moves angularly on the front surface in the direction opposite the first direction, for example, to the right.

Throughout the document, movement to the left or to the right, or upwards or downwards, shall be understood to mean a movement seen from the point of view of the user handling the rupturing tool.

First, the bilateral rupturing tool of the invention allows carving a straight bone tunnel when the rupturing element is in a first position in which its axis of rupture is coaxial with the main longitudinal axis of the rupturing tool. Then, the bilateral rupturing tool allows the user to widen the intraarticular outlet opening of said bone tunnel when the rupturing element is in a second position after having described an angular movement, such that it is oriented according to a second longitudinal axis forming an angle α with respect to the main longitudinal axis.

This intraarticular outlet opening will have a geometry similar to the geometry of the original anatomical area of insertion of the ligament or tendon being repaired. For example, an elongated intraarticular outlet opening having a funnel-shaped and/or rectangular geometry can be carved in respective tibial and femoral bone tunnels for restoring an ACL in the knee joint, and a likewise elongated intraarticular outlet opening having a funnel-shaped and/or rectangular geometry can be carved in a transhumeral bone tunnel for repairing a damaged supraspinatus tendon in the shoulder joint.

In a first particular example of use of the tool for repairing a damaged supraspinatus muscle tendon of the rotator cuff, the dimensions of the intraarticular outlet opening of the bone tunnel to be created must be such that they allow the insertion of the end of the damaged tendon which, at the height of the rotator chord, has measurements of between 4 mm and 5 mm in thickness and between 20 mm and 25 mm in width.

In a second particular example of use of the tool for reconstructing an anterior cruciate ligament, the intraarticular outlet opening of the bone tunnel to be created must be such that it allows the insertion of the fibrous substance, having measurements of between 2 and 4 mm in thickness and between 12 mm and 18 mm in width.

However, these particular measurement ranges provided in the two particular examples of use must be adapted based on the anatomy of each patient and on the final application for which the rupturing tool is used which, in a general use, is suitable for rupturing any connective tissue and/or cartilage tissue and/or bone tissue, in medicine or veterinary science.

Advantageously, such anatomical widenings created with the tool greatly favor tissue healing, which means that the operation is effective and its result is long-lasting.

In another particular example of use, the rupturing tool is suitable for creating a space of selected shape and dimensions by means of the efficient rupture and elimination of tissue from the intervertebral disk, during minimally invasive vertebral discectomies.

In a particular example of use, the rupturing tool is suitable for rupturing any connective tissue and/or cartilage tissue and/or bone tissue, in medicine or veterinary science.

In a particular example of use, the rupturing tool is suitable for creating and/or carving all types of tunnels in surgical interventions for the reconstruction of any connective tissue and/or cartilage tissue and/or bone tissue, in medicine or veterinary science.

In one embodiment, the rupturing tool is disposable.

In a particular embodiment, *the rupturing assembly goes through and is fixed to the inside of the through hole:*
- *by means of the rupturing element; or*
- *by means of the movement transfer element; or*
- *partially by means of the rupturing element and partially by means of the movement transfer element; or*
- *by means of a bearing fitted in the through hole; or*
- *by means of a combination of two or more of the foregoing.*

In a particular embodiment, the rupturing assembly goes through and is fixed to the inside of the through hole by means of a bushing or bearing fitted in the through hole.

The attachment between the movement transfer element and the rupturing element can be proximal with respect to the through conduit, or distal with respect to the through conduit, or said attachment can be located in the through conduit.

In this particular embodiment, different possible configurations between the rupturing assembly and the articulated attachment means are contemplated, notwithstanding the probable existence of other configurations which are viable in the context of the invention. As set forth above, the articulated attachment means comprises a central section with a through hole with the rupturing assembly going through same, said rupturing assembly being fixed to the inside of the through hole.

In one embodiment, the element which goes through and is fixed to the through hole is the rupturing element, which is securely attached to the movement transfer element on the outside of the through hole.

In one embodiment, the element which goes through and is fixed to the through hole is the movement transfer element, which is securely attached to the rupturing element on the outside of the through hole.

In one embodiment, the rupturing element and the movement transfer element each partially goes through the through hole and is fixed to the inside thereof. In this case, the attachment between said elements is established inside the through hole.

In this embodiment, different possible configurations between the rupturing assembly and the articulated attachment means are contemplated, notwithstanding the probable existence of other configurations which are viable in the context of the invention. As set forth above, the articulated attachment means comprises a central section with a through hole with the rupturing assembly going through same, said rupturing assembly being fixed to the inside of the through hole.

In one embodiment, the element which goes through and is fixed to the through hole is the rupturing element, which is attached to the movement transfer element securely or by a fixing means, on the outside of the through hole.

In one embodiment, the element which goes through and is fixed to the through hole is the movement transfer element, which is attached to the rupturing element securely or by a fixing means, on the outside of the through hole.

In one embodiment, both the rupturing element and the movement transfer element each partially goes through the through hole and is fixed to the inside thereof. In this case, the attachment between said elements is established inside the through hole.

In one embodiment, the element which goes through and is fixed to the through hole is a bearing which serves as a fixing means between the movement transfer element and the rupturing element.

In a particular embodiment, *the movement transfer element and*/*or the rupturing element are cannulated.*

Throughout the document, the adjective "*cannulated"* when it refers to an element shall be understood to mean that said element comprises a longitudinal conduit going through same.

In a particular embodiment, *the bilateral rupturing tool further comprises at least a first attachment and*/*or coupling means configured for attaching and*/*or coupling the movement transfer element to a rupturing movement generator device.*

The bilateral rupturing tool can be coupled to a rupturing movement generator device; for example, a drilling device generating a rotary movement. The tool, particularly the movement transfer element, receives the rupturing movement from said rupturing movement generator device and in turn transfers it to the rupturing element.

In anticipation of the probable existence of many types of different rupturing movement generator devices, the option of the tool comprising one or more attachment and/or coupling means configured for attaching or coupling the movement transfer element to one or more of said devices is contemplated in this embodiment.

Advantageously, the tool has even greater versatility, where it can be coupled to any rupturing movement generator device which may be commercially available.

In a particular embodiment, *the bilateral rupturing tool further comprises a support body at least partially surrounding the longitudinal element, and a second attachment and*/*or coupling means configured for attaching and*/*or coupling the support body to a rupturing movement generator device.*

In this embodiment, the tool comprises a support body, being understood to be a body which makes it easier to grip the tool, partially surrounding the longitudinal element. Optionally, this body can furthermore be attached or coupled to a rupturing movement generator device by one or more second attachment and/or coupling means

In anticipation of the probable existence of many types of different rupturing movement generator devices, the option of the tool comprising one or more second attachment and/or coupling means configured for attaching or coupling the support body to one or more of said devices is contemplated in this embodiment.

Advantageously, the tool has even greater versatility, where it can be coupled to any rupturing movement generator device which may be commercially available.

In a particular embodiment, *the bilateral rupturing tool further comprises a rupturing guide configured for guiding the longitudinal element from an original position to at least one target position, wherein the rupturing guide comprises:*
- *a tubular guide comprising:*
   ∘ *a longitudinal conduit with a proximal end and a third distal end; the longitudinal conduit comprising a distal appendage at the third distal end; and*
   ∘ *a striking edge, a prolongation of the longitudinal conduit at the proximal end; and*

   *wherein the longitudinal conduit is configured for housing therein the longitudinal element, and*
   *wherein the tubular guide is oriented according to a first longitudinal guiding axis; and*
   *the striking edge is configured for receiving a striking force in the direction of the first longitudinal guiding axis; and*
- *a guide arch comprising:*
   ∘ *a proximal arch portion comprising a first coupling means configured for securely coupling and uncoupling the proximal arch portion with respect to the tubular guide, in a plurality of different positions along the tubular guide,*
   ∘ *a distal arch portion in turn comprising a distal tip and a distal point through which the first longitudinal guiding axis passes; and*
   ∘ a second coupling means configured forc oupling and uncoupling the distal arch portion with respect to the proximal arch portion in a plurality of different positions, such that starting from a first position according to which the tubular guide is oriented according to a first longitudinal guiding axis, when the second coupling means is in a position other than the first position according to a second longitudinal guiding axis, it forms an angle β with the first longitudinal axis.

In this embodiment, the tool contemplates a rupturing guide configured for guiding the rupturing element and the longitudinal element from a first original position, that in which the rupturing element is located coaxially to the main longitudinal axis (to start carving the straight bone tunnel), to at least a first target position, in particular, that in which the rupturing element has moved forward linearly carving the straight bone tunnel. Additionally, the rupturing guide is configured for guiding the rupturing element and the longitudinal element from a second original position, that in which the rupturing element is angled with respect to the main longitudinal axis (having widened the intraarticular outlet opening of the straight bone tunnel), to at least a second target position, in particular, that in which the user has caused the rupturing tool to move backwards with the rupturing element being angled with respect to the longitudinal axis (for thereby retrocarving the intraarticular outlet opening of the initially carved straight bone tunnel).

Advantageously, the use of a rupturing guide allows increasing precision in the placement of the rupturing tool for carving the bone tunnel in the target anatomical position, as well as for improving the actual usability of the tool.

First, the rupturing guide comprises at least one tubular guide. Said guide in turn comprises the following elements:
- A longitudinal conduit, preferably tubular, with a proximal end and a distal end. This conduit is sized to enable housing the longitudinal element therein. Furthermore, the distal end comprises a distal appendage (for example, a peripheral recess), being understood to be a distal end portion, a prolongation of the longitudinal conduit, the cross-section of which has a diameter smaller than the diameter of the rest of the longitudinal conduit. In a particular embodiment, this distal appendage comprises polyhedral faces.
- A striking edge, a prolongation of the longitudinal conduit at the proximal end. In the context of the present invention, "striking edge" shall be understood to mean a projection of the longitudinal conduit at the proximal end thereof. For example, the striking edge can be a part adhered to the longitudinal assembly or integrated in said longitudinal assembly as a single part.

The guide is oriented according to the mentioned first longitudinal guiding axis, which will coincide with the main longitudinal axis according to which the longitudinal element is oriented when it is inserted in the rupturing guide.

The method of introducing the rupturing tool comprises the following steps:
- carving the entire bone tunnel by means of the rupturing tool and the rupturing guide;
- removing the guide arch by maintaining the rupturing tool inside the bone tunnel and inside the tubular guide; and
- introducing the distal appendage in a first fragment of the bone tunnel by striking the striking edge.

The distal appendage is sized and configured for being inserted in the first fragment of the bone tunnel once carved. The diameter thereof is such that the introduction of this appendage in the first fragment of the bone tunnel with certain tightening is allowed and the introduction of the rest of the longitudinal conduit in said bone tunnel is prevented. Preferably, the outer diameter of the appendage is substantially equal to the diameter of the first fragment of the bone tunnel or slightly larger.

To introduce said appendage in the first fragment of the bone tunnel, the user must exert a specific striking force on the proximal end of the striking edge in the direction of the first longitudinal guiding axis. This force can be applied, for example, by means of a hammer.

Preferably, the proximal end of the striking edge is substantially flat.

Advantageously, the force applied on the striking edge ensures a play-free penetration of the distal appendage in the first fragment of the bone tunnel. This allows the guide to remain in a stable position, which increases precision in the placement of the longitudinal element forc arving the second segment of the bone tunnel in the target anatomical position, as well as for improving the actual usability of the tool.

Second, the rupturing guide comprises a guide arch. As such, a curved element must be understood to comprise a proximal arch portion which in turn comprises first coupling means which allow coupling said proximal arch portion securely to the tubular guide, in a plurality of different positions along same. The guide arch further comprises a distal arch portion ending in a distal tip and comprising a distal point through which the first longitudinal guiding axis passes. Lastly, the guide arch comprises a second coupling means which allows coupling this distal arch portion to the proximal arch portion in a plurality of different positions.

The first longitudinal guiding axis according to which the tubular guide is oriented when the second coupling means is in one position forms a different angle α2 with the first longitudinal guiding axis according to which the tubular guide is oriented when the second coupling means is in any of the other positions.

Preferably, the distal tip of the distal portion is configured forb eing introduced in the patient's body and thereby fixing the rupturing guide. In one example, the distal portion has an elliptical, cylindrical, or annular configuration, and optionally, the distal tip is sharp.

The fixing of the distal tip in the patient's body advantageously allows achieving the more stable guide position, which allows increasing precision in the placement of the longitudinal element for carving the bone tunnel in the target anatomical position, as well as for improving the actual usability of the tool.

Moreover, the first coupling means is configured for securely coupling and uncoupling the proximal arch portion with respect to the tubular guide in a plurality of different positions along the guide. Advantageously, this first coupling means allows positioning the tubular guide in different positions, making the rupturing guide highly versatile and enabling the adaptation thereof to a specific application (medicine or veterinary science), to a particular surgery, and/or to the specific anatomy of the patient. Another additional advantage is that the guide arch and the tubular guide can easily uncoupled, which makes it easier to remove the guide arch once the longitudinal element has been inserted in the tubular guide and the bone tunnel has been carved.

The second coupling means between the proximal arch portion and the distal arch portion allows the guide to be adapted to the direction in which the user wishes to carve the first segment of the bone tunnel based on their preferences and on the anatomy of each patient.

Widening of the intraarticular outlet opening of the bone tunnel requires the rupturing element to be angled to both sides of the first longitudinal guiding axis, with the rupturing movement activated.

In a particular embodiment, *the tubular guide further comprises a first longitudinal hole and a second longitudinal hole;*
*the first longitudinal body further comprises a first projection; and*
*the second longitudinal body further comprises a second projection;*
*wherein the first longitudinal hole is sized and configured for receiving and guiding the first projection from a proximal location to a distal location, and vice versa; and*
*the second longitudinal hole is sized and configured for receiving and guiding the second projection from a proximal location to a distal location, and vice versa.*

In this embodiment, the invention contemplates two holes in the guide and two projections in the longitudinal element, located in the first longitudinal body and the second longitudinal body. Each hole-projection pair seeks to guide the rupturing element both for creating the straight bone tunnel and for carving the intraarticular outlet opening.

Both holes are sized and configured for receiving and guiding both projections of the longitudinal bodies from a proximal location, that is, from the point at which one of the longitudinal holes receives its corresponding projection, to a distal location, in which said projection penetrates the corresponding longitudinal hole all the way to the bottom. Since the longitudinal bodies move in opposite directions, when the projection of the first longitudinal body is in a proximal location, the projection of the second longitudinal body is in a distal location; and vice versa.

Furthermore, the hole-projection pairs also allow guiding the rupturing element during a retrocarving procedure. Therefore, once the straight bone tunnel is created, the user can cause the rupturing tool to move backwards inside the tubular guide in the distal-proximal direction in order to widen said intraarticular outlet opening. The rupturing movement must be kept activated in this widening.

Advantageously, the rupturing guide increases the usability and precision of the rupturing tool when creating a widened intraarticular outlet opening of the bone tunnel.

In a particular embodiment, *the distal appendage comprises:*
- *a trilobular tip; or*
- *two tips; or*
- *a beveled recess; or*
- *polyhedral faces.*

In this embodiment, different types of distal appendage are provided, notwithstanding the probable existence of other types which fall within the context of the invention.

In a particular embodiment, *the bilateral rupturing tool further comprises an actuator configured for generating and transmitting a linear movement to the first longitudinal body and to the second longitudinal body such that both longitudinal bodies move linearly in opposite directions.*

Therefore, the movement of the two longitudinal bodies is coordinated by the actuator: when one of the longitudinal bodies moves in one direction, the other longitudinal body moves linearly in the opposite direction. Both linear movements are simultaneous movements in opposite directions and are driven by the actuator.

Advantageously, the actuator allows a user of the rupturing tool to be able to angle the rupturing element at will. Furthermore, for the rupturing element to perform the angular movement, the longitudinal bodies of the longitudinal element must move linearly in opposite directions.

In this embodiment, widening of the bone tunnel using the bilateral rupturing tool is carried out by means of the actuator, such that the user angles the rupturing element to one side of the first longitudinal guiding axis and retrocarves a first widening segment of the bone tunnel. Then, again by means of the actuator, the user angles the rupturing element in the opposite direction and retrocarves a second widening segment of the bone tunnel.

In particular, in different particular embodiments of the rupturing tool of the invention, the rupturing element can be located in different positions:
a) coaxial to the first longitudinal axis (it is used in this position to carve the straight bone tunnel);
b) angled to a first side of the first longitudinal axis; and
c) angled to a second side of the first longitudinal axis, with the second side being opposite the first side.

In one embodiment, *the first longitudinal body comprises a first longitudinal notch configured for cooperating with the actuator; and the second longitudinal body comprises a second longitudinal notch configured for cooperating with the actuator;*
*and wherein the actuator comprises:*
- *a first rod configured for penetrating and cooperating with the first longitudinal notch;*
- *a second rod configured for penetrating and cooperating with the second longitudinal notch; and*
- *an actuation trigger attached to the first rod;*
   *wherein the first rod and the second rod are arranged in parallel and attached to one another by two longitudinal parts at the ends thereof such that:*
   - *when the actuation trigger performs a rotational movement in the proximal-distal direction with respect to an axis of rotation located between the first rod and the second rod,*
      *the first rod performs a rotational movement in the proximal-distal direction, causing the first longitudinal body to perform a proximal-distal linear movement; and*
      *the second rod performs a rotational movement in the distal-proximal direction, causing the second longitudinal body to perform a distal-proximal linear movement; and*
   - *when the actuation trigger performs a rotational movement in the distal-proximal direction with respect to the axis of rotation,*
      *the first rod performs a rotational movement in the distal-proximal direction, causing the first longitudinal body to perform a distal-proximal linear movement; and*
      *the second rod performs a rotational movement in the proximal-distal direction, causing the second longitudinal body to perform a proximal-distal linear movement.*

This embodiment describes a first embodiment of an actuator configured for generating and transmitting a linear movement to the longitudinal element, with which it cooperates mechanically.

On one hand, the first longitudinal body and the second longitudinal body of the longitudinal element each comprises a longitudinal notch; both longitudinal notches being configured for cooperating with the actuator.

The actuator in turn comprises a first rod and a second rod, each of which is sized and configured for penetrating the notches of the longitudinal element and cooperating with same. Said rods are arranged in parallel and attached to one another by two longitudinal parts, such that the two rods and the two longitudinal parts form a quadrilateral. Additionally, the actuator comprises a manual trigger, which can be operated by the user, attached to the first rod.

When the user operates the actuation trigger, it describes a movement in the proximal-distal direction or distal-proximal direction. The attachment of said trigger with the first rod causes the quadrilateral formed by the rods and the longitudinal parts to in turn describe a rotation with respect to its own axis of rotation, which will be located between the first rod and the second rod.

If the movement described by the trigger is in the proximal-distal direction, the first rod performs a rotational movement with respect to the axis of rotation of the quadrilateral in the proximal-distal direction, causing the first longitudinal body to perform a proximal-distal linear movement. In turn, the second rod performs a rotational movement in the opposite direction, that is, the distal-proximal direction, causing the second longitudinal body to perform a distal-proximal linear movement.

In contrast, if the movement described by the trigger is in the distal-proximal direction, the first rod performs a rotational movement with respect to the axis of rotation of the quadrilateral in the distal-proximal direction, causing the first longitudinal body to perform a distal-proximal linear movement. In turn, the second rod performs a rotational movement in the opposite direction, that is, the proximal-distal direction, causing the second longitudinal body to perform a proximal-distal linear movement.

Advantageously, this mechanism allows the longitudinal bodies of the longitudinal element to move in a synchronized manner in opposite directions with the actuation trigger, in turn causing the rupturing element to describe an angular movement in both directions. In other words, the actuator of this embodiment, by means of this "elongated hole" or groove geometry, successfully transforms a rotational movement of the trigger into a linear movement of the longitudinal element.

In one embodiment, *the bilateral rupturing tool further comprises positioning and securing means for the actuator, wherein the positioning and securing means for the actuator are configured for positioning and securing the actuator in a plurality of different positions, such that, for each of said positions, the distance between the first distal end and the second distal end of both longitudinal bodies is different.*

In this embodiment, it is contemplated that the tool has positioning and securing means for the actuator, which are configured for positioning said actuator in different positions and securing said actuator fixed in the selected position.

Preferably, the positioning and securing means for the actuator position and secure the actuator in three positions:
1. a first end position in which the first longitudinal body achieves its maximum proximal-distal movement and the second longitudinal body achieves its maximum distal-proximal movement;
2. a second end position in which the first longitudinal body achieves its maximum distal-proximal movement and the second longitudinal body achieves its maximum proximal-distal movement; and
3. a neutral position in which both longitudinal bodies are aligned, such that the relative distance between their distal ends is substantially zero.

Alternatively, besides the two end positions and the neutral position, the positioning and securing means for the actuator allow positioning and securing the actuator in other intermediate positions.

In a more particular embodiment, the positioning and securing means fort he actuator comprise a hole in the support body sized and configured for housing a ball and a spring. The spring is located at the bottom of the hole of the support body, such that the spring pushes the ball towards the outside of the hole; additionally, the trigger comprises two or more holes sized and configured for partially housing the ball.

When the hole of the support body is arranged facing one of the holes of the trigger, the ball, pushed by the spring, is located partially in the hole of the trigger, fixing the position. This position can be overcome by rotating the trigger, such that the ball is again housed completely inside the hole of the support body until reaching a new position in which the hole of the support body is arranged facing a new hole of the trigger, thereby allowing the positioning means to fix the different positions of the trigger in which the hole of the support body is arranged facing one of the holes of the trigger.

In one embodiment, *the actuator further comprises a return spring configured for exerting a return force on the first longitudinal body and the second longitudinal body such that the relative movement between said bodies is substantially zero.*

In this embodiment, it is contemplated for all the actuators of the preceding embodiments to comprise a return spring configured for positioning the longitudinal bodies in a neutral position, i.e., that in which the relative movement between the longitudinal bodies is zero.

In another embodiment, *the first longitudinal body comprises a first outer thread configured for cooperating with the actuator; and the second longitudinal body comprises a second outer thread configured for cooperating with the actuator; wherein the first outer thread and the second outer thread have opposite thread directions;*
*and wherein the actuator comprises*
   - *a nut which in turn comprises therein:*
      o *a first inner thread portion, matching the first outer thread; and*
      o a *second inner thread portion, matching the second outer thread; wherein the first inner thread portion and the second inner thread portion have opposite thread directions;*
*wherein the nut is configured for cooperating with the first longitudinal body and the second longitudinal body such that the assembly works like a double spindle mechanism;*
   - *an actuation control attached to the nut;*
*wherein*
   - *when the actuation control performs a rotational movement in the thread direction of the first inner thread portion, the first longitudinal body performs a proximal-distal linear movement and the second longitudinal body performs a distal-proximal linear movement; and*
   - *when the actuation control performs a rotational movement in the thread direction of the second inner thread portion, the first longitudinal body performs a distal-proximal linear movement and the second longitudinal body performs a proximal-distal linear movement.*

Throughout the document, "*spindle mechanism"* shall be understood to mean an assembly formed by a spindle screw and a nut, wherein the rotary movement of the screw when the nut is kept fixed causes a linear movement of the screw with respect to the nut and vice versa. Therefore, the rotary movement of the screw is successfully transformed into a rectilinear movement of the nut and vice versa by means of this system.

In this embodiment, a "double spindle mechanism" is used. On one hand, the first longitudinal body and the second longitudinal body each comprises an outer thread, such that they will act as spindle screws. The threads of both longitudinal bodies have contrary or opposite thread directions.

On the other hand, the actuator comprises a nut with two different inner thread portions. The first inner thread portion matches the outer thread of the first longitudinal body, whereas the second inner thread portion matches the outer thread of the second longitudinal body. Being matching shall be understood to mean that the longitudinal body can be screwed onto said thread portion. Furthermore, the first thread portion and the second thread portion have opposite thread directions.

The actuator further comprises an actuation control which is attached to the nut. The operation of this control, that is, the rotation thereof to one side or another, causes the rotation of the nut itself attached thereto. In this way, when the actuation control performs a rotation, the double spindle mechanism goes into operation, causing the longitudinal bodies, which act like spindle screws, to move linearly in different directions with respect to the nut.

Therefore, when the actuation control performs a rotation in the thread direction of the first inner thread portion, the first longitudinal body performs a proximal-distal linear movement and the second longitudinal body performs a distal-proximal linear movement. In contrast, when the actuation control performs a rotation in the thread direction of the second inner thread portion, the first longitudinal body performs a distal-proximal linear movement and the second longitudinal body performs a proximal-distal linear movement.

In one embodiment, *the bilateral rupturing tool further comprises a suction line couplable to an external suction device for the removal of tissues.*

In a particular embodiment, the rupturing tool comprises a suction line couplable to an external suction device for the removal of ruptured tissue. Preferably, the rupturing element and the rupturing movement transmission element are cannulated and comprise, each of them, a distal suction window for suctioning bone dust simultaneously with bone carving and/or for suctioning soft tissue simultaneously with tissue rupturing or reaming. In an even more particular embodiment, the external suction device comprises a vacuum system.

In one embodiment, *the first longitudinal body and*/*or the second longitudinal body are rigid and*/*or straight.*

In an embodiment, the first longitudinal body and the second longitudinal body are structural elements; i.e. they form part of the external structure of the rupturing tool to provide it with a certain resistance and rigidity. Preferably, the elements are straight and rigid to provide the rupturing tool with the appropriate rigidity for carving bone tissue efficiently.

Additionally, a second inventive aspect of the invention provides *a bilateral rupturing system for minimally invasive surgical interventions, comprising:*
- *a rupturing movement generator device configured for generating and performing a rupturing movement;*
- *at least one rupturing tool according to any of the preceding embodiments, couplable to the rupturing movement generator device at least by means of the movement transfer element;*
*and wherein the rupturing movement generator device is further configured for transferring the rupturing movement to the movement transfer element.*

In this embodiment of the bilateral rupturing system, and preferably, the actuator is comprised in the rupturing tool itself. The movement transfer element works like a mechanical coupling for transferring the rupturing movement generated by the rupturing movement generator device to the rupturing tool.

In this case, the invention contemplates not only the bilateral rupturing tool, but also said tool along with a rupturing movement generator device.

In one embodiment, the rupturing tool of the system is disposable. In another alternative or additional embodiment, at least a part of the rupturing movement generator device is disposable.

The advantages set forth above for the tool can be extrapolated to this system.

A third inventive aspect of the invention alternatively provides a *bilateral rupturing system for minimally invasive surgical interventions comprising:*
- *a rupturing movement generator device configured for generating and performing a rupturing movement;*
- *at least one rupturing tool as described above in the first inventive aspect, couplable to the rupturing movement generator device by means of the movement transfer element and by means of the support body; and wherein*
*the rupturing movement generator device further comprises an actuator configured for generating and transmitting a linear movement to the first longitudinal body and to the second longitudinal body such that both longitudinal bodies move linearly in opposite directions upon receiving the linear movement from the actuator.*

In this embodiment, the invention contemplates not only the bilateral rupturing tool, but also said tool along with a rupturing movement generator device.

In the context of the present invention, where it is not explicitly indicated that the actuator is comprised in the rupturing movement generator device, actuators refer to elements belonging to the rupturing tool itself.

In order for the rupturing element to perform the angular movement, the longitudinal bodies of the longitudinal element must move linearly in opposite directions. Therefore, this embodiment contemplates that the rupturing movement generator device provides rupturing movement to the movement transfer element so that it in turn transfers said movement to the rupturing element, and provides linear movement to the longitudinal bodies, causing said longitudinal bodies to move linearly in opposite directions.

In one embodiment, the rupturing tool of the system is disposable. In another alternative or additional embodiment, at least a part of the rupturing movement and linear movement generator device is disposable.

In one embodiment, all the rupturing systems of the invention are one-piece systems.

All the advantages derived from the rupturing tool of the first inventive aspect are applicable to any of the rupturing systems comprising said rupturing tool.

All the features and/or the steps of the methods described in this specification (including the claims, description, and drawings) can be combined in any combination, except for combinations of such mutually exclusive features.

### DESCRIPTION OF THE DRAWINGS

These and other features and advantages of the invention will be shown more clearly based on the following detailed description of a preferred embodiment, provided solely by way of illustrative and non-limiting example in reference to the attached figures.
Figures 1a-1e show different views of the bilateral rupturing tool according to an embodiment of the invention. In particular, Figure 1d shows an exploded view of the bilateral rupturing tool when it comprises a first exemplary actuator. Figure 1e corresponds to different configurations for attaching the flexible transmission element to the rupturing element.
Figure 2 shows exemplary configurations of the rupturing element according to different embodiments of the invention.
Figures 3a-3d show the bilateral rupturing tool according to an embodiment of the invention in which the tool comprises a rupturing guide.
Figures 4a-4c show side section views of said tool for different states of the first exemplary actuator.
Figures 5a-5c show, in detail, the movement of the rupturing element as a result of the action of the first exemplary actuator. Figures 5d and 5e show, respectively, a general, three-dimensional, perspective view and a side section view of the bilateral rupturing tool according to an embodiment of the invention.
Figure 6 shows, in detail, the positioning and securing means for the first exemplary actuator.
In Figures 7a-7b, Figure 7a shows an exploded view of the rupturing tool when it comprises a second exemplary actuator. Figure 7b shows a side section view of the second exemplary actuator.
Figures 8a-8c show side section views of the rupturing tool for different states of the second exemplary actuator.
Figures 9a-9f show two rupturing systems for surgical interventions in which the bilateral rupturing tool can be coupled to an orthopedic drill.
In Figures 10a-10c, Figure 10a shows the cylindrical geometry of a tunnel retrocarved with a conventional unilateral rupturing tool. Figures 10b and 10c show the funnel-shaped geometry of respective bone tunnels carved by means of the bilateral rupturing tool of the present invention.
In Figures 11a-11f, Figure 11a illustrates the steps of a method forcarving a bone tunnel with a widened intraarticular outlet opening using the system of the invention. Figures 11b-11f show a first example of use of the system for repairing the supraspinatus tendon of the shoulder joint.
Figures 12a-12f illustrate the steps of an example of use of the system for restoring an anterior cruciate ligament of the knee.
Figures 13a-13c show different configurations of implants for the anatomical restoration of the anterior cruciate ligament of the knee.

### DETAILED DESCRIPTION OF THE INVENTION

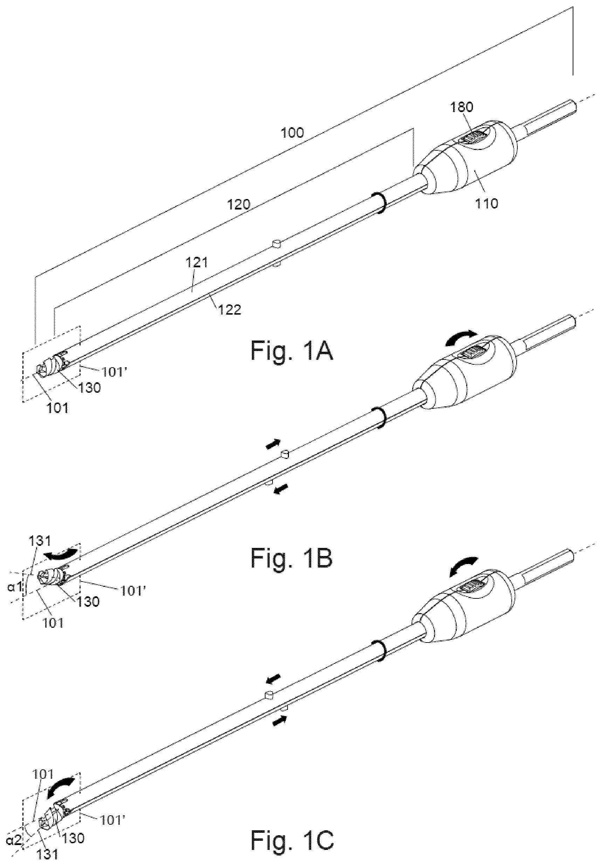

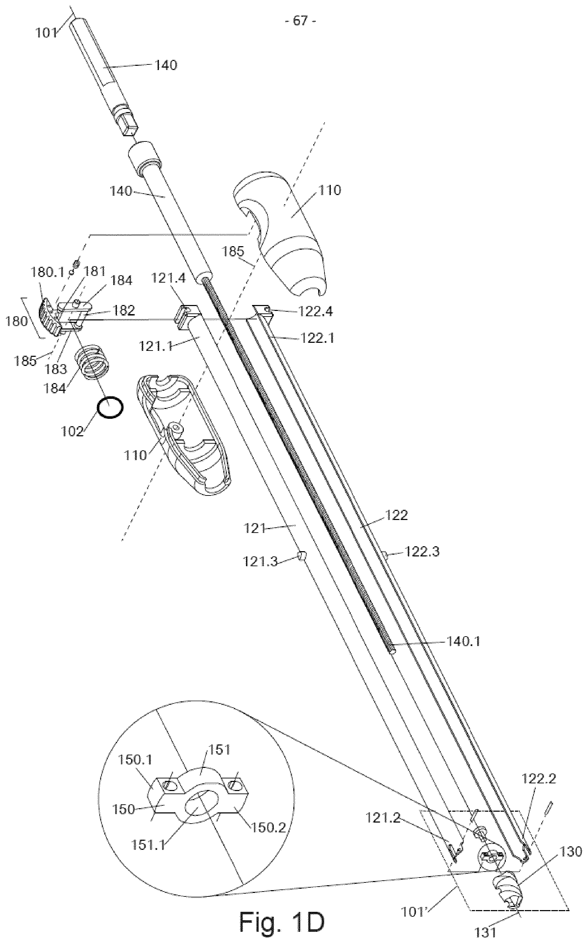

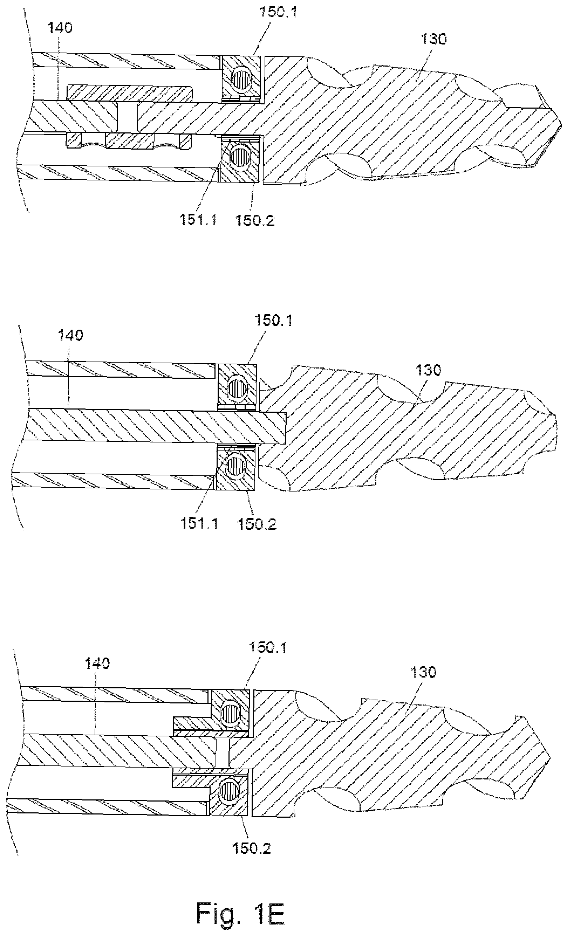

Figures 1a-1c show external perspective views of the bilateral rupturing tool (100) with the rupturing element (130) in a neutral position (Figure 1a), angled in one direction (Figure 1b), and angled in the opposite direction (Figure 1c). Figure 1d shows an exploded view of the bilateral rupturing tool (100). Figure 1e shows different configurations for attaching a movement transfer element (140) (flexible transmission element) to the rupturing element (130).

Figure 1a shows a preferred embodiment of the bilateral rupturing tool (100) for minimally invasive surgical interventions comprising a longitudinal element (120) oriented according to a main longitudinal axis (101) which in turn comprises a first longitudinal body (121) and second longitudinal body (122). Preferably, the first longitudinal body and the second longitudinal body (121, 122) are in the form of a channel, conduit, or semi-cylindrical tube, with the hollow portions of each of said bodies facing one another.

This figure further illustrates that the tool comprises an actuator (180) configured for generating and transmitting a linear movement to the first longitudinal body (121) and to the second longitudinal body (122) such that both longitudinal bodies (121, 122) move linearly in opposite directions. This actuator (180) is merely an illustrative example, where other types of similar actuators or elements performing the same function as this actuator in the context of the invention may exist.

This figure further shows a support body (110) partially surrounding the longitudinal element (120) and containing the actuator (180) therein, although said support body (110) is optional. Furthermore, the bilateral rupturing tool (100) may also optionally comprise a second attachment and/or coupling means (170) configured for attaching and/or coupling the support body (110) to a rupturing movement generator device (200, 200'), for example, a drilling device.

Second, the tool comprises a rupturing assembly. Said assembly in turn comprises a rupturing element (130) and a movement transfer element (140), not visible in this Figure 1a, attached to one another. This attachment can be a fixed attachment, such that the assembly is a one-piece assembly, or can be done by means of an intermediate attachment element, for example by means of a bearing. Furthermore, this attachment is established between the distal portion (140.1) of the movement transfer element (140) and the most proximal end of the rupturing element (130), as illustrated in Figure 1e.

The movement transfer element (140) is arranged inside the longitudinal element (120), between the first longitudinal body (121) and the second longitudinal body (122) and is configured forr eceiving and performing a rupturing movement and for transferring the rupturing movement to the rupturing element (130). Preferably, the movement transfer element (140) receives the rupturing movement from a rupturing movement generator device (200, 200'), for example, a drilling device.

In turn, the rupturing element (130) is configured for receiving the rupturing movement from the movement transfer element (140) and for performing said movement about its axis of rupture (131).

Additionally, the rupturing element (130) is configured for receiving and performing an angular movement on a front surface, such that said rupturing element (130) gradually forms a plurality of angles α with respect to the main longitudinal axis (101). In order for this rupturing element (130) to be able to perform the mentioned rupturing movement, the movement transfer element (140) is arranged inside the longitudinal element (120), that is, between the first longitudinal body (121) and the second longitudinal body (122). Preferably, the first longitudinal body and the second longitudinal body (121, 122) are semi-cylindrical tubes and the movement transfer element (140) is housed inside the hollow portion of both semi-cylindrical tubes.

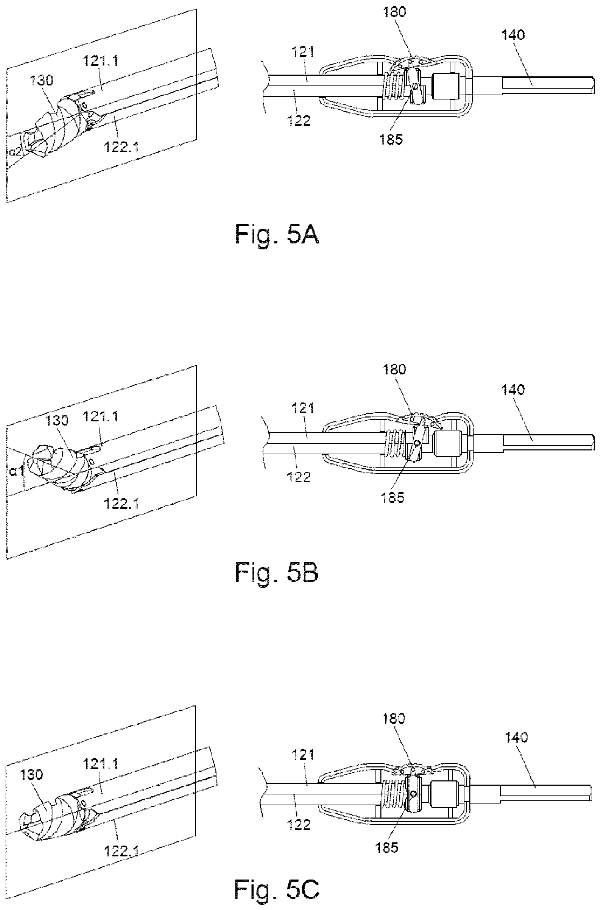

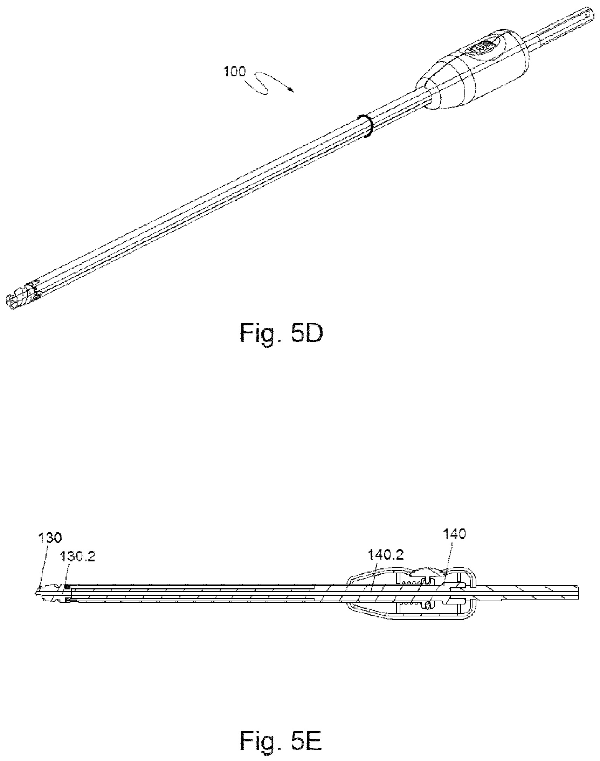

Optionally, the movement transfer element (140) and/or the rupturing element (130) are cannulated, as illustrated in detail in Figures 5d and 5e, which correspond respectively to a three-dimensional view and to a side section view of an embodiment of the rupturing tool (100) in which both the movement transfer element (140) and the rupturing element (130) comprise respective longitudinal conduits (130.2, 140.2) going through same.

Optionally, the bilateral rupturing tool (100) also comprises at least a first attachment and/or coupling means (160) configured fora ttaching and/or coupling the movement transfer element (140) to a rupturing movement generator device (200) without a second actuator (180'), for example, a drilling device; or alternatively, the movement transfer element (140) can be coupled to a rupturing movement generator device (200') comprising a second actuator (180'), this latter case not being included in Figure 1.

In a preferred embodiment, the bilateral rupturing tool (100) comprises an actuator (180, 190) and at least a first attachment and/or coupling means (160) configured for attaching and/or coupling the movement transfer element (140) to a rupturing movement generator device (200) without an actuator, for example, a drilling device.

In another particular embodiment, the bilateral rupturing tool (100) does not comprise the actuator (180, 190) and instead comprises attachment and/or coupling means (160, 170) for attaching and/or coupling to a rupturing movement generator device (200) which in turn comprises an actuator (180', 190').

Figures 1b and 1c show by means of arrows the movement described by the elements of the tool (100) in use. On one hand, both longitudinal bodies (121, 122), which are arranged in parallel, perform respective linear movements along the main longitudinal axis (101). The direction of movement depends, respectively, on the rotation performed by the user on the trigger (180.1) of the actuator (180). The operation of this actuator (180) will be explained in detail in later figures.

Similarly, the direction of rotation of the rupturing element (130) depends on the direction of the linear movements of the longitudinal bodies (121, 122).

Preferably, the movement transfer element (140) is in the form of a rod or an elongated cylinder.

Lastly, the tool (100) comprises an articulated attachment means (150), partially visible in Figures 1a-1c, which is formed by three sections attached to one another forming a single part:
- a first section (150.1) which is in turn attached to the first longitudinal body (121) at the distal end (121.1) thereof;
- a second section (150.2) which is in turn attached to the second longitudinal body (122) at the distal end (122.1) thereof; and
- a central section (151) arranged between the first section (150.1) and the second section (150.2), comprising a through hole (151.1) with the rupturing assembly going through same, and said rupturing assembly being fixed to the inside of the through hole (151.1).

Preferably, the attachment between the first section (150.1) and the first longitudinal body (121) as well as the attachment between the second section (150.2) and the second longitudinal body (122) is a mechanical attachment, for example by means of hinges. Alternatively, this attachment or these attachments can be flexible, that is, all the elements form a one-piece assembly but their attachments maintain degrees of freedom.

The articulated attachment means (150) is configured for performing an angular movement about the through hole (151.1) in the main plane of the tool (100), this angular movement being the result of the linear movements of the first longitudinal body and the second longitudinal body (121, 122). In that sense:
- on one hand, when the first longitudinal body (121) performs linear movement in the proximal-distal direction, the first section (150.1) of the articulated attachment means (150) also describes a proximal-distal movement. Simultaneously, the second longitudinal body (122) performs linear movement in the distal-proximal direction and the second section (150.2) of the articulated attachment means (150) also describes a distal-proximal movement. In this way, both sections (150.1, 150.2) move in opposite directions about the through hole (151.1) of the central section (151).
- On the other hand, when the first longitudinal body (121) performs linear movement in the distal-proximal direction, the first section (150.1) describes a distal-proximal movement and, simultaneously, the second longitudinal body (122) and the second section (150.2) of the articulated attachment means (150) perform the corresponding linear movement in the proximal-distal direction.

The purpose of this angular movement is to cause the rupturing element (130) to perform angular movement to the left or right (or upwards-downwards, according to how the tool is positioned) with respect to the main longitudinal axis (101) in the main plane. Since the rupturing assembly goes through and is fixed to the inside of the through hole (151.1) of the attachment means (150), the angular movement of said attachment means (150) causes the angular movement of the rupturing element (130). In particular, when the first section (150.1) performs a proximal-distal movement, the rupturing element (130) moves angularly on the front surface in a first direction, for example, to the left (or downwards); whereas when the second section (150.2) is the one which performs said proximal-distal movement, the rupturing element (130) moves angularly on the front surface in the direction opposite the first direction, for example, to the right (or upwards).

Figure 1d shows an exploded view of all the elements of the rupturing tool (100) according to the embodiment shown in Figures 1a-1c. It should be borne in mind that the actuator (180) is one of several possible actuators in the context of the invention, being shown merely as an illustrative example.

A reference ring (102) surrounding the longitudinal element (120), where said ring can move along the element (120), can furthermore be seen in these Figures 1a-1d. This ring (102), which is completely optional, is used as a reference for the user who is using the rupturing tool (100), in particular, it is used to inform the user of the depth of retrocarving.

Figure 1e shows, in detail, different alternatives of the configuration existing between the articulated attachment means (150) and the rupturing assembly. Said rupturing assembly goes through and is fixed to the inside of the through hole (151.1):
- by means of the rupturing element (130), which is attached to the movement transfer element (140) on the outside of the through hole (151.1); or
- by means of the movement transfer element (140), which is attached to the rupturing element (130) on the outside of the through hole (151.1); or
- partially by means of the rupturing element (130) and partially by means of the movement transfer element (140), such that the attachment between said elements (130, 140) is established in this case inside the through hole (151.1).

In all these examples, the rupturing assembly goes through and is fixed to the inside of the through hole (151.1) by means of a bushing or a bearing fitted in the through hole (151.1).

In one embodiment, the bilateral rupturing tool (100) further comprises a suction line couplable to an external suction device for the removal of tissues. Preferably, the rupturing element (130) is cannulated and comprises a distal suction window for suctioning bone dust simultaneously with bone carving and/or for suctioning soft tissue simultaneously with tissue rupturing or reaming. In an even more particular embodiment, the external suction device comprises a vacuum system.

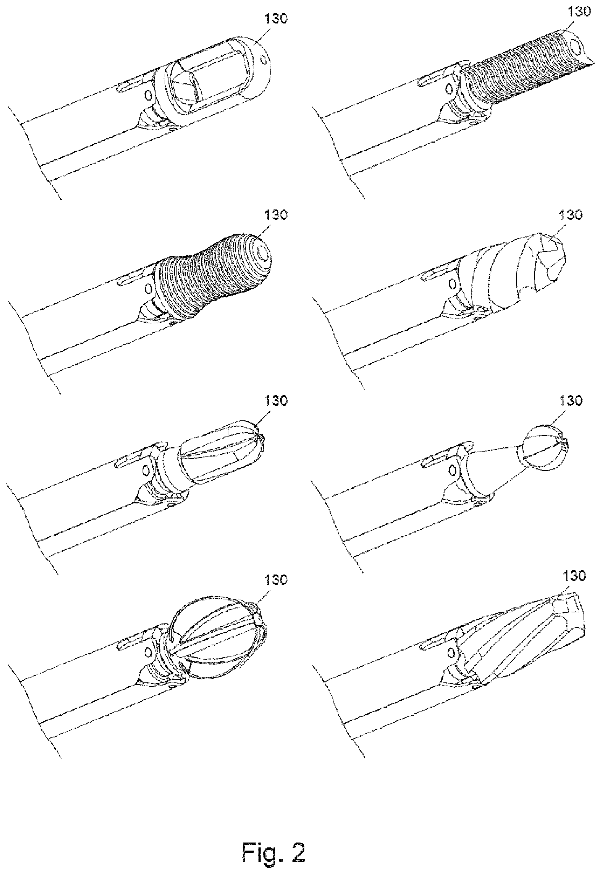

Figure 2 shows exemplary configurations of the rupturing element (130) according to different embodiments of the invention.

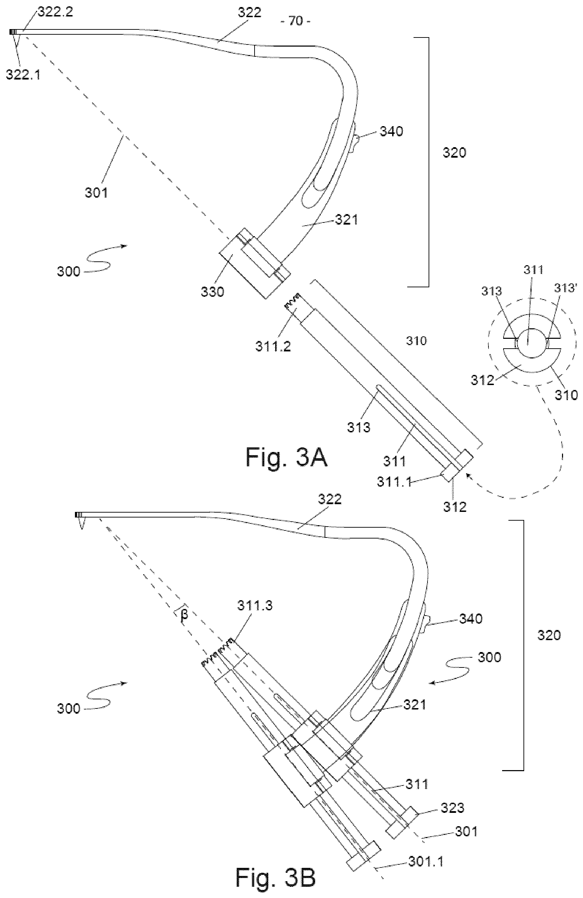

Figures 3a-3d show the bilateral rupturing tool (100) according to an embodiment of the invention in which the tool (100) comprises a rupturing guide (300) which is configured for guiding the longitudinal element (120) from an original position to at least one target position.

First, the rupturing guide (300) comprises at least one tubular guide (310). Said guide (310) in turn comprises the following elements:
- a preferably tubular longitudinal conduit (311) with a third proximal end (311.1) and a distal end (311.2). This conduit (311) is sized to enable housing therein the longitudinal element (120). The distal end (311.2) further comprises a distal appendage (311.3), for example, a peripheral recess, a prolongation of the longitudinal conduit (311), the cross-section of which has a diameter smaller than the diameter of the rest of the longitudinal conduit (311).
- A striking edge (312), a prolongation of the longitudinal conduit (311) at the third proximal end (311.1).

The tubular guide (310) is oriented according to a first longitudinal guiding axis (301), which will coincide with the main longitudinal axis (101) according to which the longitudinal element (120) is oriented when it is inserted in the rupturing guide (300).

The distal appendage (311.2) is sized and configured for being inserted in a first fragment of the bone tunnel once carved. Some examples of the distal appendage (311.3) comprise a trilobular tip, or two tips, or a beveled recess, or polyhedral faces.

To introduce said appendage (311.2) in the first fragment of the bone tunnel, the user must exert a specific striking force on the proximal end of the striking edge (312) in the direction of the first longitudinal guiding axis (301).

Preferably, the proximal end of the striking edge (312) is substantially flat, as can be seen in these figures.

Second, the rupturing guide (300) comprises a guide arch (320) comprising a proximal arch portion (321) which in turn comprises a first coupling means (330) which allows securely coupling said proximal arch portion (321) to the tubular guide (310) in a plurality of different positions along same. The guide arch (320) further comprises a distal arch portion (322) ending in a distal tip (322.1) and comprising a distal point (322.2) through which the first longitudinal guiding axis (301) passes. Lastly, the guide arch comprises a second coupling means (340) which allows coupling and uncoupling this distal arch portion (322) with respect to the proximal arch portion (321) in a plurality of different positions.

Starting from a first position according to which the tubular guide (320) is oriented according to a first longitudinal guiding axis (301), when the second coupling means (340) is in a position other than the first position according to a second longitudinal guiding axis (301.1), it forms an angle β with the first longitudinal axis (301).

The distal tip (322.1) is configured for being introduced in the patient's body and thereby fixing the rupturing guide, said distal tip being sharp in these particular figures.

The first coupling means (330) is configured for securely coupling and uncoupling the proximal arch portion (321) with respect to the tubular guide (310) in a plurality of different positions along the guide. These elements are uncoupled in Figure 3a, while being coupled in Figure 3b.

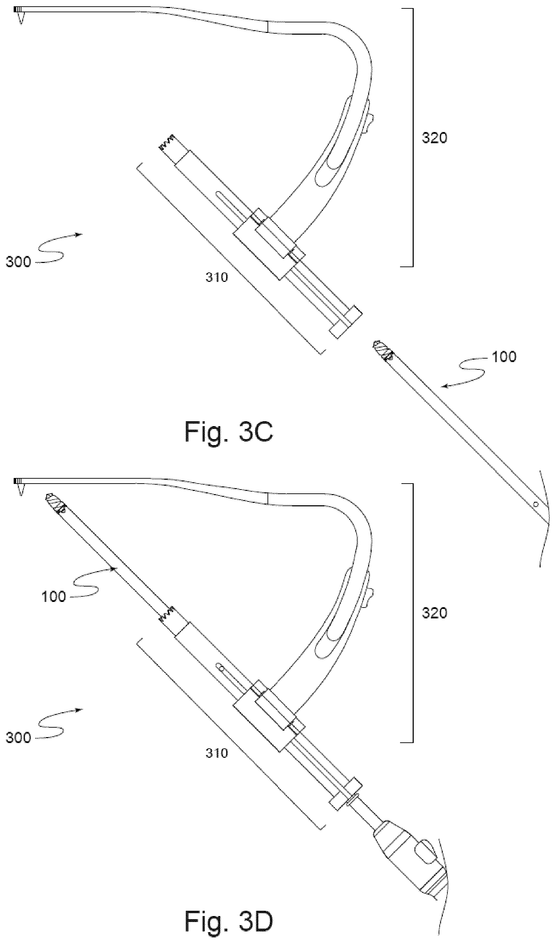

Moreover, Figure 3c shows the rupturing guide (300) in which the longitudinal element (120) of the tool (100) will be introduced and Figure 3d shows the tool (100) when said longitudinal element (120) has been introduced all the way to the bottom of the rupturing guide (300).

Lastly, although optional, Figures 3a-3d show that the tubular guide (310) further comprises a first longitudinal hole (313) and a second longitudinal hole (313'), which are sized and configured for receiving, respectively, a first projection (121.3) located in the first longitudinal body (121) and a second projection (122.3) located in the second longitudinal body (122). In this way, the longitudinal holes (313, 313') allow guiding the first projection (121.3) and the second projection (122.3) from a proximal location to a distal location, and vice versa.

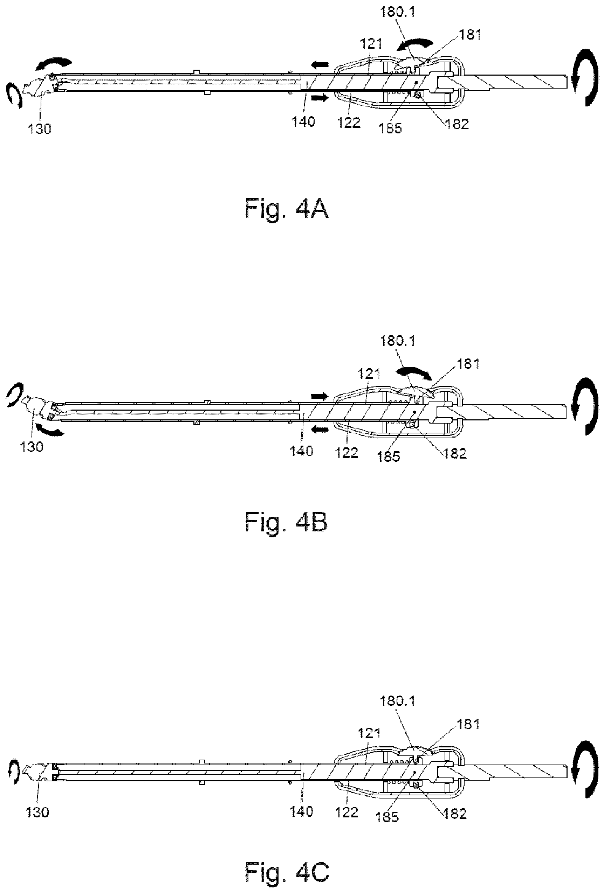

Going back to Figure 1d, this figure shows an exploded view of the rupturing tool (100) when it comprises a first exemplary actuator (180). Figures 4a-4c show side section views of said tool (100) for different states of the actuator (180). This actuator (180) is configured for generating and transmitting a linear movement to the first longitudinal body (121) and to the second longitudinal body (122) such that both longitudinal bodies (121, 122) move linearly in opposite directions.

To that end, the first longitudinal body (121) comprises a first longitudinal notch (121.4) configured for cooperating with the actuator (180), and the second longitudinal body (122) comprises a second longitudinal notch (122.4) configured for cooperating with the actuator (180). These notches can be seen in detail in Figure 1d, showing the tool with this actuator (180) in an exploded view.

In turn, the actuator (180) comprises:
- a first rod (181) configured for penetrating and cooperating with the first longitudinal notch (121.4);
- a second rod (182) configured forp enetrating and cooperating with the second longitudinal notch (122.4); and
- an actuation trigger (180.1) attached to the first rod (181).

The first rod (181) and the second rod (182) are arranged in parallel and attached to one another by two longitudinal parts (183, 184) at the ends thereof such that, together, they form a quadrilateral. In turn, the actuation trigger (180.1) is a manual trigger, which can be operated by the user.

When the user operates the actuation trigger (180.1), it can perform a rotational movement in the proximal-distal direction or distal-proximal direction. The attachment of said trigger (180.1) with the first rod (181) causes the quadrilateral formed by the rods (181, 182) and the longitudinal parts (183, 184) to in turn describe a rotation with respect to its own axis of rotation (185), which will be located between the first rod (181) and the second rod (182).

Figure 4a shows an example in which the movement described by the trigger (180.1) is in the proximal-distal direction. With this movement, the first rod (181) in turn performs a rotational movement with respect to the axis of rotation (185) of the quadrilateral in the proximal-distal direction, causing the first longitudinal body (121) to perform a proximal-distal linear movement. In turn, the second rod (182) performs a rotational movement in the opposite direction, that is the distal-proximal direction, causing the second longitudinal body (122) to perform a distal-proximal linear movement. As can be seen in the figure, these movements of the longitudinal bodies (121, 122) cause the rupturing element (130) to describe an angular movement in a first direction, in this case downwards or to the left (according to how the rupturing tool has been positioned).

Figure 4b shows an example in which the movement described by the trigger (180.1) is in the distal-proximal direction. With this movement, the first rod (181) in turn performs a rotational movement with respect to the axis of rotation (185) of the quadrilateral in the distal-proximal direction, causing the first longitudinal body (121) to perform a distal-proximal linear movement. In turn, the second rod (182) performs a rotational movement in the opposite direction, that is, the proximal-distal direction, causing the second longitudinal body (122) to perform a proximal-distal linear movement. As can be seen in the figure, these movements of the longitudinal bodies (121, 122) cause the rupturing element (130) to describe an angular movement in the direction opposite the first direction, in this case upwards or to the right (according to how the rupturing tool has been positioned).

Figure 4c shows an example in which the trigger (180.1) has not been moved in any direction, that is, it is in an intermediate or neutral position. In such case, linear movement of the longitudinal bodies (121, 122) does not occur, so the rupturing element (130) remains in a neutral position in which it remains coaxial to the longitudinal element (120).

Lastly, it should be mentioned that Figures 4a-4c show at the same time the rupturing movement received by the movement transfer element (140), in this case a rotational movement, which it transmits to the rupturing element (130).

Figures 5a-5c show, in detail, the movement of the rupturing element (130) as a result of the action of the actuator (180) according to the three states described in Figures 4a-4c, respectively.

Figures 5a-5c show, in detail, a particular exemplary positioning and securing means for the first exemplary actuator (180). Both in this example and in other possible examples, these positioning and securing means for the actuator (180) are configured for positioning and securing the actuator (180) in a plurality of different positions, such that for each of said positions, the distance between the first distal end (121.1) and the second distal end (122.1) of both longitudinal bodies (121, 122) is different.

Preferably, the positioning and securing means for the actuator position and secure the actuator (180) in at least three positions:
1. a first end position in which the first longitudinal body (121) achieves its maximum proximal-distal movement and the second longitudinal body (122) achieves its maximum distal-proximal movement;
2. a second end position in which the first longitudinal body (121) achieves its maximum distal-proximal movement and the second longitudinal body (122) achieves its maximum proximal-distal movement; and
3. a neutral position in which both longitudinal bodies (121, 122) are aligned, such that the relative distance between their distal ends (121.1, 122.1) is substantially zero.

Alternatively, besides the two end positions and the neutral position, the positioning and securing means for the actuator allow positioning and securing the actuator (180) in other intermediate positions.

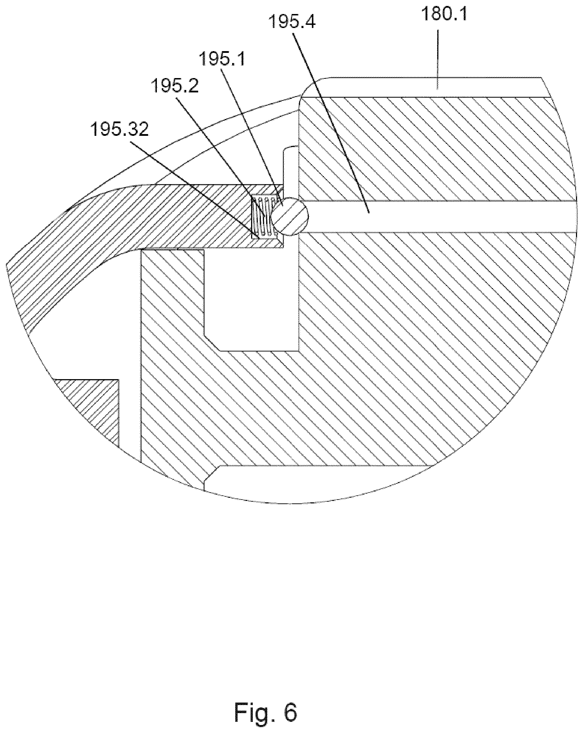

In the particular example of Figure 6, the positioning and securing means for the actuator (180) comprise a hole (195.3) in the support body (110) sized and configured for housing a ball (195.1) and a spring (195.2). The spring (195.2) is located at the bottom of the hole (195.3) of the support body (110), such that the spring (195.2) pushes the ball (195.3) towards the outside of the hole (195.3); the trigger (180.1) further comprises two or more holes (195.4) sized and configured for partially housing the ball (195.1).

When the hole (195.3) of the support body (110) is arranged facing one of the holes (195.4) of the trigger (180.1), the ball (195.3), pushed by the spring (195.2), is located partially in the hole of the trigger (180.1), fixing a position. This position can be overcome by rotating the trigger (180.1), such that the ball (195.1) is again housed completely inside the hole (195.3) of the support body (110) until reaching a new position in which the hole (195.3) of the support body (110) is arranged facing a new hole (195.4) of the trigger (180.1), thereby allowing the positioning means to fix the different positions of the trigger (180.1) in which the hole (195.4) of the support body (110) is arranged facing one of the holes (195.4) of the trigger (180.1).

In one embodiment, the actuator (180) further comprises a return spring configured for exerting a return force on the first longitudinal body and the second longitudinal body (121, 122) such that the relative movement between said bodies (121, 122) is substantially zero. Therefore, this return spring is configured for positioning the longitudinal bodies (121, 122) in a neutral position, i.e., that in which none of the longitudinal bodies (121, 122) is arranged in a forward or rearward position with respect to the other one.

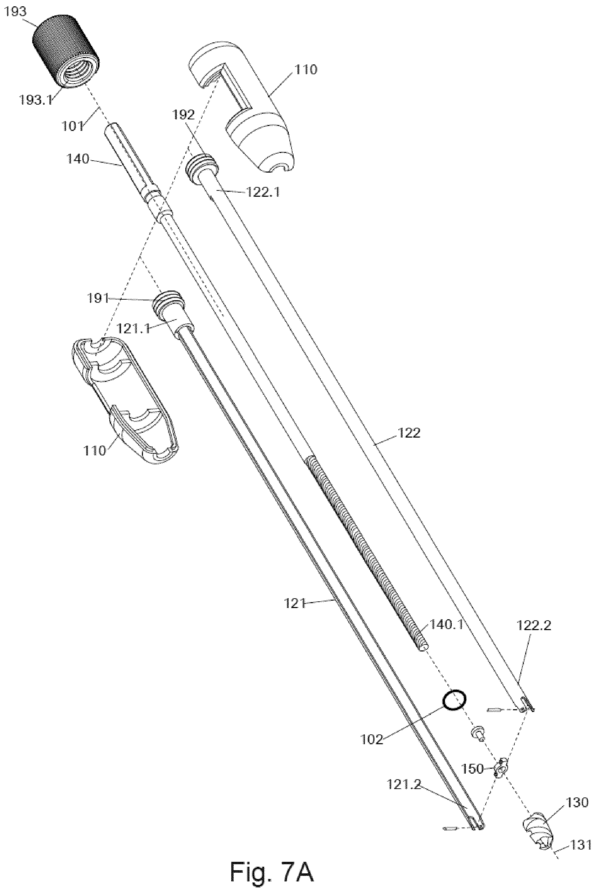

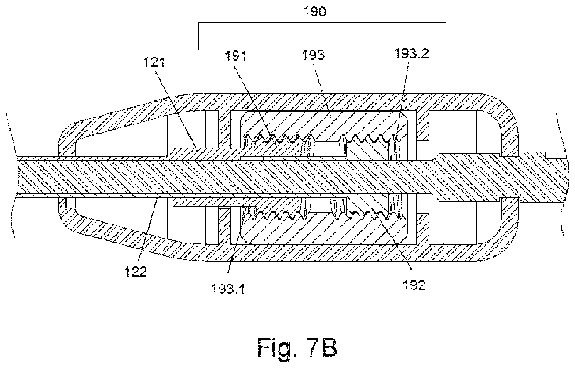

Figure 7a shows an exploded view of the rupturing tool (100) when it comprises a second exemplary actuator (190). Figure 7b shows a side section view of the second exemplary actuator (190).

This actuator (190) is configured for generating and transmitting a linear movement to the first longitudinal body (121) and to the second longitudinal body (122) such that both longitudinal bodies (121, 122) move linearly in opposite directions.

To that end, the first longitudinal body (121) comprises a first outer thread (191) configured for cooperating with the actuator (190) and the second longitudinal body (122) comprises a second outer thread (192) configured for cooperating with the actuator (190). These first outer thread (191) and second outer thread (192) have opposite thread directions and can be seen in detail in Figure 7a, showing the tool (100) with this actuator (190) in an exploded view.

The actuator (190) comprises a nut (193) which in turn comprises therein a first inner thread portion (193.1), matching the first outer thread (191), and a second inner thread portion (193.2), matching the second outer thread (192). The first inner thread portion (193.1) and the second inner thread portion (193.2) have opposite thread directions and the nut (193) is configured for cooperating with the first longitudinal body (121) and the second longitudinal body (122) such that the assembly works like a double spindle mechanism.

On one hand, the outer threads (191, 192) of the first longitudinal body and the second longitudinal body (121, 122) cause these bodies to act like spindle screws. On the other hand, the inner threads (193.1, 193.2) of the nut (193) interact with said spindle screws such that one and the same nut (193) performs a double function. As a result of the opposite thread direction in both inner threads (193.1, 193.2), when the nut (193) rotates, one of the spindle screws moves linearly in one direction and the other moves linearly in the opposite direction, as will be seen in detail below.

The actuator (190) further comprises an actuation control (194) which is attached to the nut (193). The operation of this control (194), that is, the rotation thereof to one side or another, causes the rotation of the nut (193) itself attached thereto. In this way, when the actuation control (194) performs a rotation, the double spindle mechanism goes into operation causing the longitudinal bodies (121, 122), which act like spindle screws, to move linearly in opposite directions.

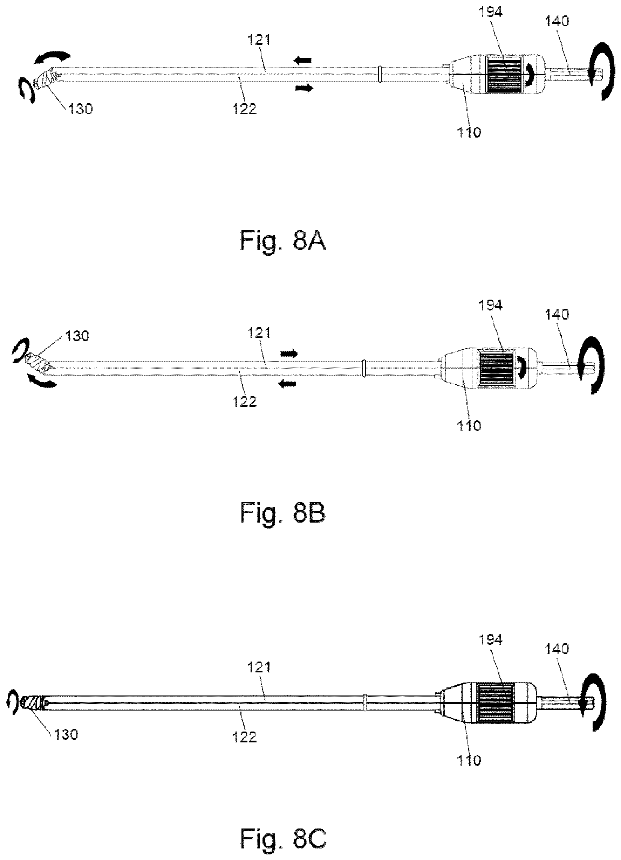

Figures 8a-8c show side section views of the rupturing tool (100) for different states of the second exemplary actuator (190).

Figure 8a shows an example in which the actuation control (194) performs a rotation in the thread direction of the first inner thread portion (193.1). This causes the first longitudinal body (121) to perform a proximal-distal linear movement and the second longitudinal body (122) to perform a distal-proximal linear movement. As can be seen in Figure 8a, these movements of the longitudinal bodies (121, 122) cause the rupturing element (130) to describe an angular movement in a first direction, in this case downwards or to the left (according to how the rupturing tool has been positioned).

In contrast, as shown in Figure 8b, when the actuation control (194) performs a rotation in the thread direction of the second inner thread portion (193.2), the first longitudinal body (121) performs a distal-proximal linear movement and the second longitudinal body (122) performs a proximal-distal linear movement. As can be seen in Figure 8b, these movements of the longitudinal bodies (121, 122) cause the rupturing element (130) to describe an angular movement in the direction opposite the first direction, in this case upwards or to the right (according to how the rupturing tool has been positioned).

Figure 8c shows an example in which the control (194) has not been moved in any direction, that is, it is in an intermediate or neutral position. In such case, linear movement of the longitudinal bodies (121, 122) does not occur, so the rupturing element (130) remains in a neutral position in which it remains coaxial to the longitudinal element (120).

Advantageously, the actuator (190) fixes the different angulation positions by means of a reverse spindle mechanism without requiring additional positioning means.

Lastly, it should be mentioned that Figures 8a-8c show at the same time the rupturing movement received by the movement transfer element (140), in this case a rotational movement, which it transmits to the rupturing element (130).

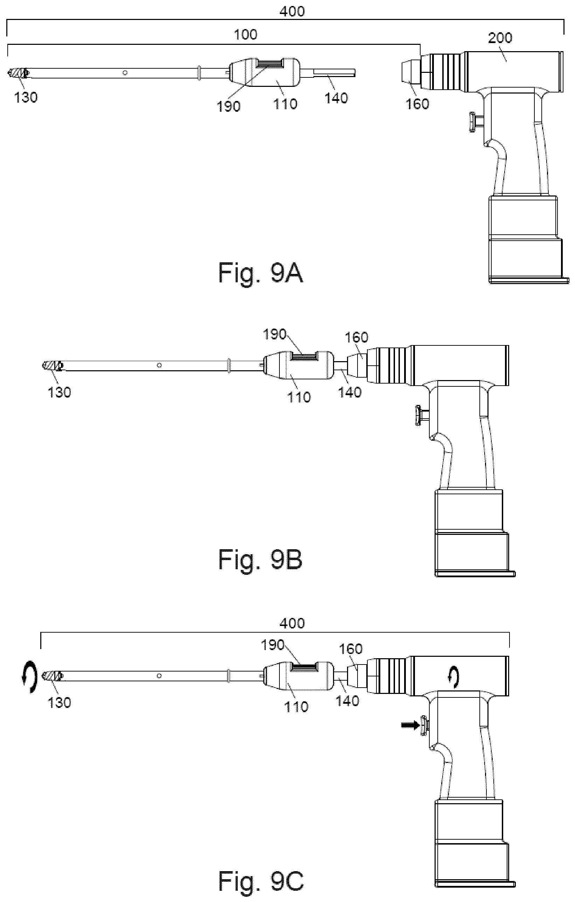

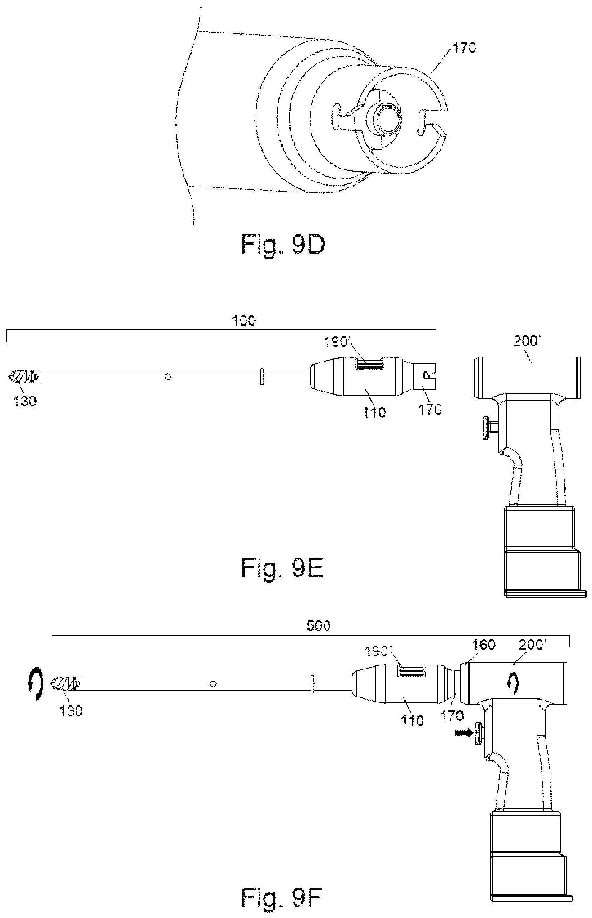

Figures 9a-9f show two embodiments of rupturing systems (400, 500) for surgical interventions. In particular, Figures 9a-9d refer to a first rupturing system (400), whereas Figures 9e-9f refer to a second rupturing system (500). The rupturing system (400) comprises a rupturing movement generator device (200) and a bilateral rupturing tool (100) with an actuator according to any of the preceding embodiments. In this rupturing system (400), the rupturing tool (100) comprises the actuator (180, 190).

Moreover, the rupturing system (500) comprises a rupturing movement generator device (200') in turn comprising an actuator (180', 190'), so in this particular embodiment the rupturing tool does not comprise an actuator; and wherein the linear movement in opposite linear directions performed by the first longitudinal body (121) and the second longitudinal body (122) is generated and transmitted by the actuator (180', 190') of the rupturing movement generator device (200').

The rupturing movement generator devices (200, 200') in these figures are drilling devices (200, 200'), which are configured for generating and performing a rupturing movement, in this particular example a rotary movement. Additionally, they are configured for transferring the rupturing movement to the movement transfer element (140).

In particular, Figure 9a shows a side view of the system (400) with the bilateral tool (100) uncoupled from the rupturing movement generator device (200).

Figure 9b shows a side view of the system (400) with the bilateral tool (100) coupled to the rupturing movement generator device (200), which is inactive. This coupling is established between the rupturing movement generator device (200) and the movement transfer element (140) through a first attachment and/or coupling means (160).

Figure 9c shows a side view of the system (400) with the bilateral tool (100) coupled to the rupturing movement generator device (200), which is inactive like in the preceding figure. In this case, the coupling is established between the rupturing movement generator device (200) and the movement transfer element (140) through a first attachment and/or coupling means (160) and between the rupturing movement generator device (200) and the support body (110) through a second attachment and/or coupling means (170).

Figure 9d shows, in detail, the second attachment and/or coupling means (170) above.

Figure 9e shows a side view of a second rupturing system (500) comprising a rupturing movement generator device (200') and a rupturing tool (100). In this Figure 9e, the rupturing movement generator device (200') is shown uncoupled from the rupturing tool (100)..

Figure 9f shows the second rupturing system (500) for surgical interventions comprising:
- the rupturing movement generator device (200') configured forg enerating and performing a rupturing movement, said rupturing movement generator device (200') being an actuator (190');
- at least one bilateral rupturing tool (100) without an actuator, couplable to the rupturing movement generator device (200') by means of the movement transfer element (140) and by means of the support body (110); wherein
the rupturing movement generator device (200') is further configured for transferring the rupturing movement to the movement transfer element (140), as well as the linear movement to the first longitudinal body (121) and the second longitudinal body (122) by means of the actuator (190'). The linear movement of the first longitudinal body (121) is in a direction opposite the direction of the second longitudinal body (122).

In this example illustrated in Figure 9f, the coupled position of the rupturing system (500) of Figure 9e is shown, in which the rupturing tool (100) is coupled to the rupturing movement generator (200'). The coupling is established between the rupturing movement generator device (200') and the movement transfer element (140) through a first attachment and/or coupling means (160), and between the rupturing movement generator device (200') and the support body (110) through a second attachment and/or coupling means (160). In the example of Figure 9f, it is indicated by means of arrows that the rupturing movement generator device is activated (arrow pointing to the trigger of the drill), which causes the rotation of the rupturing assembly.

In the particular example, the rupturing movement generator device (200') comprises mechanical and/or electronic means which are regulated at will by the user.

In a particular embodiment, the rupturing system (400, 500) comprises a disposable bilateral rupturing tool (100).

In another particular embodiment, the rupturing system (400, 500) comprises a bilateral rupturing tool (100) and a rupturing movement and linear movement generator device (200) or a rupturing movement generator device (200') which are disposable, with the exception of the motor and the battery of the rupturing movement generator device (200, 200').

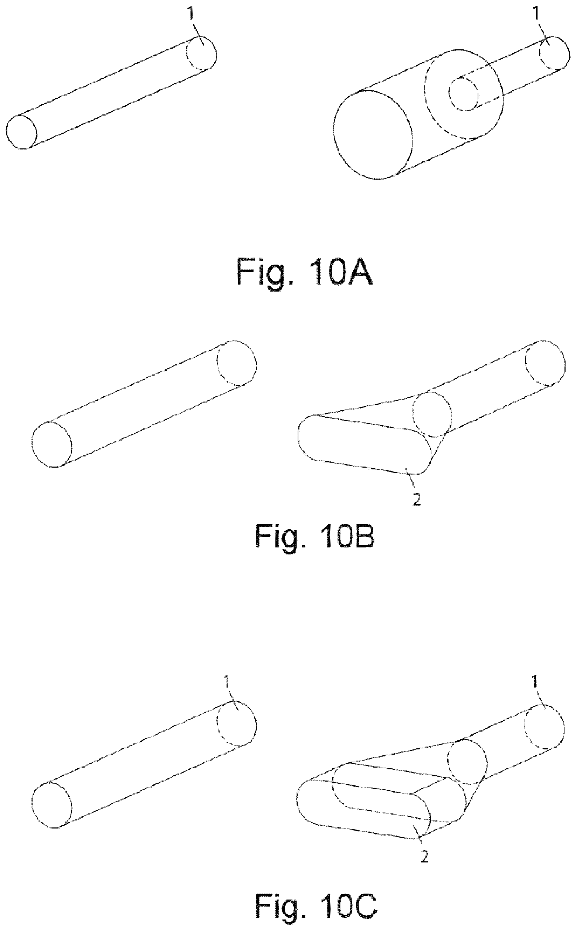

Figure 10a shows the state of the art known for retrocarving bone tunnels by means of previously known rupturing tools which have a single axis of rotation that coincides with the axis of rupture: the retrocarving only provides a cylindrical widening of the first bone tunnel.

Figure 10b shows a bone tunnel created and widened by means of the bilateral rupturing tool (100) of the present invention.

It should be pointed out that the particular embodiments of the bilateral rupturing tool (100) show the advantages of the invention with respect to rupturing tools available in the state of the art, and these advantages include the following:
- smaller number of parts, and therefore a lower manufacturing and assembly cost; and
- greater rigidity of the assembly such that it allows carving bone tissue.

### Surgical method performed by means of the rupturing system of the present invention.

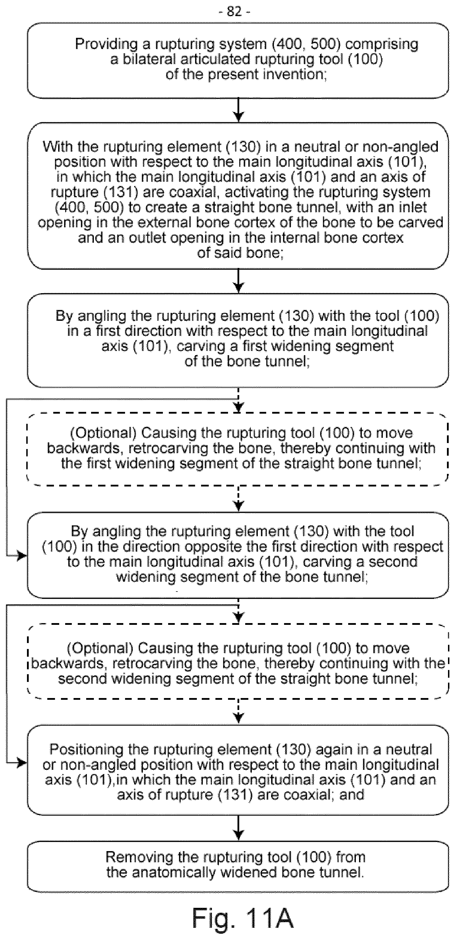

In a fourth inventive aspect, complementary to the first inventive aspect, the invention provides a method for carving a bone tunnel with a widened intraarticular outlet opening in a human or animal body during connective tissue repair. This method is illustrated in Figure 11a by means of a flow chart.

The method comprises the following steps:
a) providing a rupturing system (400, 500) comprising a bilateral articulated rupturing tool (100) of the present invention;
b) with the rupturing element (130) in a neutral or non-angled position with respect to the main longitudinal axis (101), in which the main longitudinal axis (101) and an axis of rupture (131) are coaxial, activating the rupturing system (400, 500) to create a straight bone tunnel, with an inlet opening in the external bone cortex of the bone to be carved and an outlet opening in the internal bone cortex of said bone;
c) by angling the rupturing element (130) with the tool (100) in a first direction with respect to the main longitudinal axis (101), carving a first widening segment of the bone tunnel;
d) (optional) causing the rupturing tool (100) to move backwards, retrocarving the bone, thereby continuing with the first widening segment of the straight bone tunnel;
e) by angling the rupturing element (130) with the tool (100) in the direction opposite the first direction with respect to the main longitudinal axis (101), carving a second widening segment of the bone tunnel;
f) (optional) causing the rupturing tool (100) to move backwards, retrocarving the bone, thereby continuing with the second widening segment of the straight bone tunnel;
g) positioning the rupturing element (130) again in a neutral or non-angled position with respect to the main longitudinal axis (101), in which the main longitudinal axis (101) and the axis of rupture (131) are coaxial; and
h) removing the rupturing tool (100) from the anatomically widened bone tunnel.

Throughout the description, "retrocarving" shall be understood to mean the action of causing the rupturing element (130) to move backwards from a first distal position in a bone tunnel to a second proximal position in the bone tunnel, such that by means of this action the segment of the bone tunnel travelled by the rupturing element (130) is widened.

In this example, in step c) the angle defined between the main longitudinal axis (101) and the axis of rupture (131) is referred to as α1, which is the angle defining the first widening segment of the bone tunnel. Moreover, in step e), the angle defined between the main longitudinal axis (101) and the axis of rupture (131) is referred to as α2.

To carry out the method of this example, the user can additionally provide a rupturing movement generator device (200, 200').

In other specific embodiments, the method described above can be performed without any of optional steps d) or f), or without including any of said steps.

In an alternative particular method, a unilateral articulated rupturing tool having sufficient rigidity for carving bone tissue can be used in step a). In this alternative particular method, step e) of changing the direction of angulation of the rupturing element (130) with respect to the main longitudinal axis (101) is replaced by the joint rotation of the unilateral articulated rupturing tool and the rupturing movement generator device (200, 200'). This joint rotation is technically demanding for the user, requiring practice and reducing precision in the method of carving the bone tunnel, and may even cause complications to arise, so the method is preferably performed by means of the bilateral rupturing system (400, 500) of the invention specifically designed for this task.

### First example of use of the rupturing system: repair of the supraspinatus tendon of the shoulder joint.

In a first example of use, the connective tissue on which the repair (that is, the preceding method) is carried out is the supraspinatus tendon of the shoulder joint.

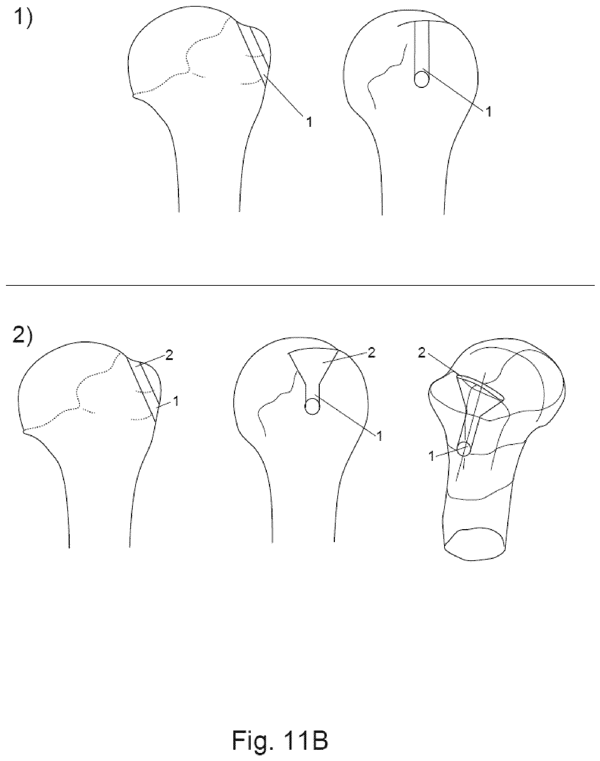

Figure 11b shows, in detail, the different segments of the bone tunnel carved in the greater tubercle of the humerus:
1) side and front views of the straight bone tunnel (1).
2) side, front, and perspective views of the complete bone tunnel (1-3) with the intraarticular outlet opening (2, 3) anatomically widened by means of the system (400, 500) of the invention.

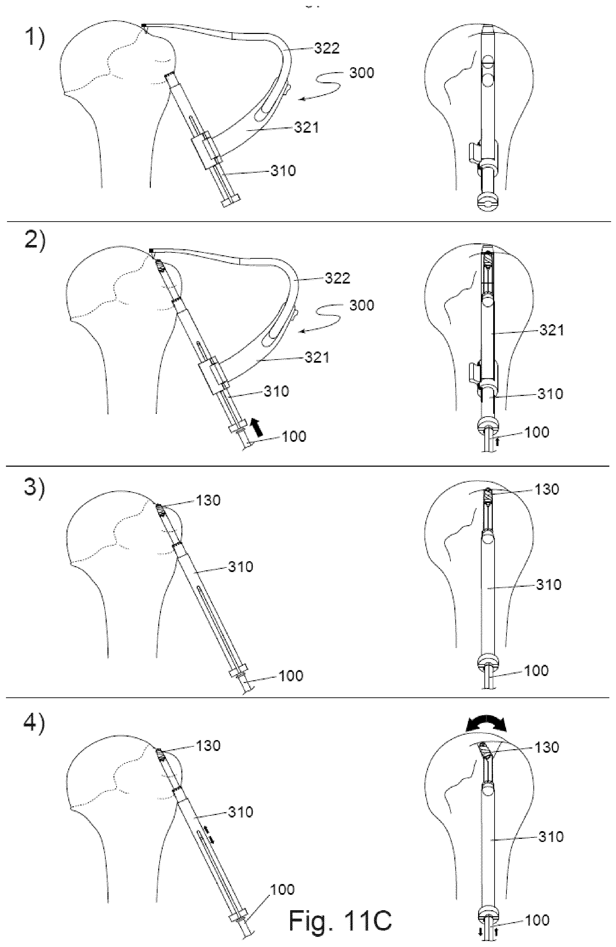

Figure 11c shows a preferred more particular embodiment in which the rupturing system (400, 500) used in the method comprises a rupturing tool (100) which in turn comprises a rupturing guide (300).

In this embodiment of the method, step b) comprises the following sub-steps:
b1) inserting the distal tip (322.1) of the distal portion (322) of the guide arch (320) of the rupturing guide (300) into the patient's body, positioning said distal tip (322.1) approximately in the center of the original insertion footprint;
b2) with the guide arch (320) parallel to the coronal plane, positioning the distal end of the tubular guide (310) of the rupturing guide (300) in the external bone cortex of the bone to be carved at an approximate distance of between 15 and 25 mm in distance from the greater tubercle;
b3) carving a straight tunnel in the bone using a bit with laser depth marking;
b4) removing the bit and applying a striking force on the striking edge (312) of the tubular guide (310) so that the distal appendage (311.3) of the tubular guide (310) penetrates the straight tunnel.

In other words, in this embodiment, the bone tunnel is carved completely (step b3) and, leaving the rupturing tool (100) inside the bone tunnel and inside the tubular guide (310), the guide arch (320) is removed and a force is applied on the striking edge (312) in order to insert the distal tip (322.1) of the tubular guide (310) in the bone tunnel.

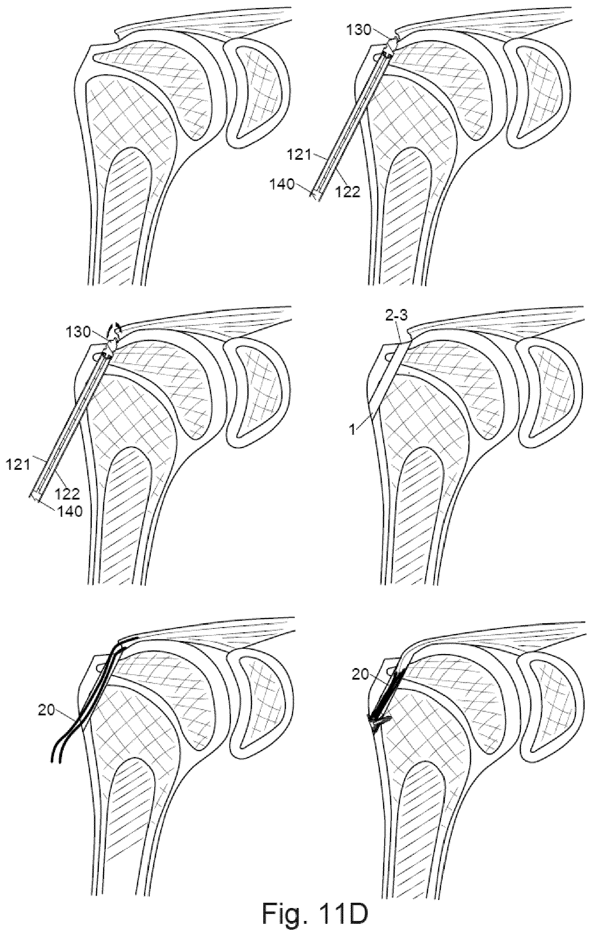

Figure 11d shows a musculoskeletal diagram of the bone tunnel (1-3) carved with one of the systems (400, 500) of the invention and the insertion of a graft forr epairing a supraspinatus muscle tendon of the shoulder joint.

In the particular example of repairing the damaged supraspinatus muscle tendon of the rotator cuff, the dimensions of the intraarticular outlet opening of the bone tunnel to be created must be such that they allow the insertion of the end of the damaged tendon which, at the height of the rotator chord, has measurements of between 4 mm and 5 mm in thickness and between 20 mm and 25 mm in width. However, these particular measurement ranges provided in the preceding examples must be adapted based on each anatomy and on the final application for which the rupturing tool is used which, in a general use, is suitable for rupturing any connective tissue and/or cartilage tissue and/or bone tissue, in medicine or veterinary science.

Once the angled bone tunnel is created and the intraarticular outlet opening widened into a fan or funnel (2-3) by means of any of the rupturing systems (400, 500) of the invention, the end of the supraspinatus muscle tendon being repaired is sutured and the suture bands are passed through the widened angled bone tunnel (1-3), for example, by means of a curved suture passer. Figure 11d shows on the bottom left part the end of the tendon which already passes through the anatomically widened opening of the bone tunnel and the suture bands which come out of the bone tunnel. Figure 11d shows on the bottom right part the suture bands retained by means of a cortical fixing device such as the one described in European patent application EP3897455 A1.

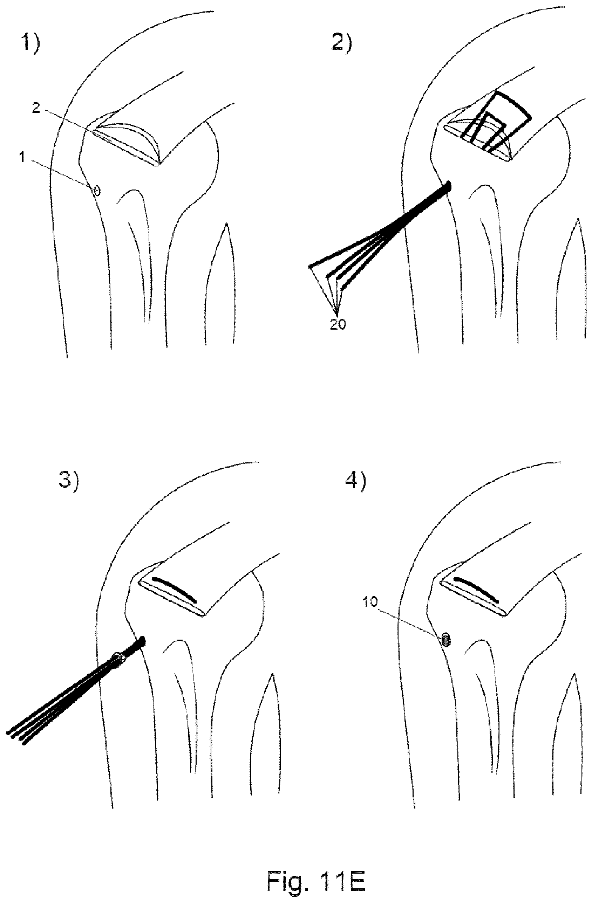

Figure 11e shows the repair of a partial supraspinatus muscle tendon rupture by means of a bone tunnel created with any of the rupturing systems (400, 500) of the invention and using suture bands (20) with which the end of the tendon is passed through the anatomically widened opening of the bone tunnel. The bottom right part of the figure shows the suture bands (20) retained by means of a cortical fixing device such as the one described European patent application EP3897455 A1.

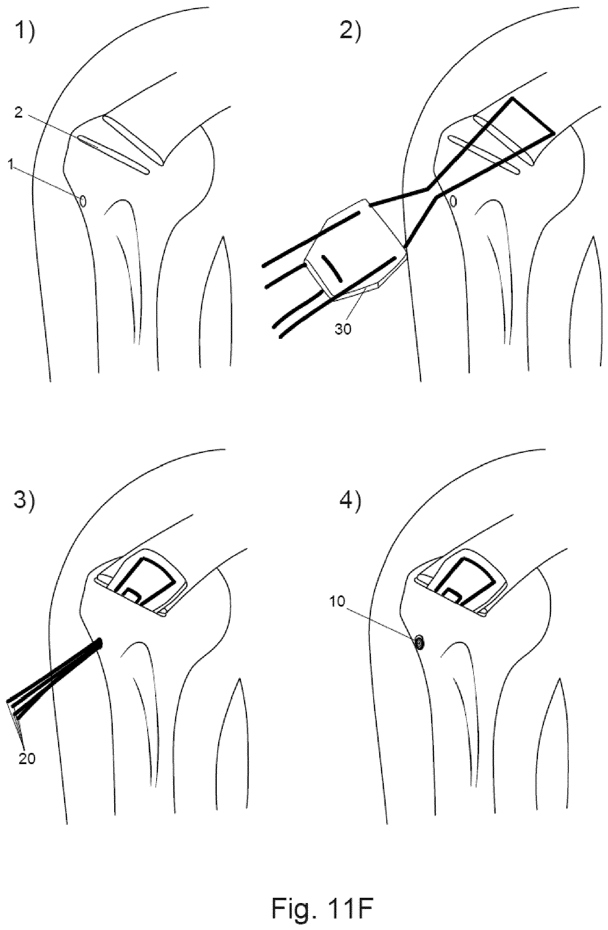

Figure 11f shows a general view of the steps of a method forr epairing a complete supraspinatus muscle tendon rupture. This method requires creating an anatomically widened bone tunnel created by means of any of the rupturing systems (400, 500) of the invention, and the use of augmentation tissue (30) and suture bands (20) with which the end of the augmentation tissue is passed through the anatomically widened opening of the bone tunnel. In a particular embodiment, the augmentation tissue (30) is a decellularized dermal tissue. In another particular embodiment, the augmentation tissue (30) comprises biodegradable biopolymer fibers. In another particular embodiment, the augmentation tissue (30) comprises a bioabsorbable poly(lactic-co-glycolic acid) (PLGA) aligned microfiber scaffold.

Taking into account that failure rates of up to 68% are reported for complete supraspinatus muscle tendon ruptures (Jost B, Pfirrmann CWA, Gerber C. Clinical outcome after structural failure of rotator cuff repairs. J Bone Joint Surg Am 2000; 82:304-14*.*), in all the cases, the objective is to achieve revascularization of the end of the tendon through the osteointegration of the augmentation tissue (30).

### Second example of use of the rupturing system: repair of the anterior cruciate ligament of the knee joint.

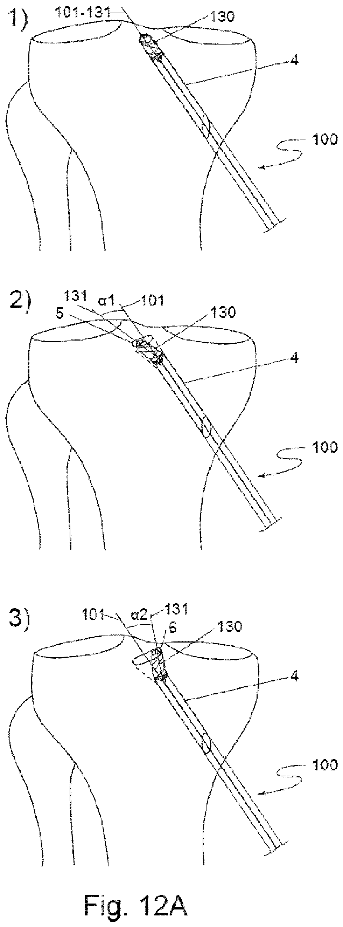

In a second example of use, the connective tissue on which the restoration is carried out is a cruciate ligament of the knee joint. Figure 12a shows the steps of the method described in Figure 11a, but performed on the tibia:
1. point 1 shows the straight bone tunnel (4) with an inlet opening in the external bone cortex of the bone and an outlet opening in the internal bone cortex of said bone, created in the tibia by means of the rupturing system (400, 500) of the invention. During this step, the axis of rupture (131) of the rupturing element (130) remains coaxial to the main longitudinal axis (101) of the rupturing tool (100);
2. point 2 shows how the rupturing element (130) is angled in a first direction with respect to the main longitudinal axis (101) of the rupturing tool (100), thereby carving a first widening segment (5) of the intraarticular outlet opening of the straight bone tunnel (4);
3. point 3 shows how the rupturing element (130) is angled in the direction opposite the first direction with respect to the main longitudinal axis (101) of the rupturing tool (100), thereby carving a second widening segment (6) of the intraarticular outlet opening of the straight bone tunnel (1).

After these actions, the axis of rupture (131) of the rupturing element (130) would move to the position in which the axis of rupture (131) is coaxial with the main longitudinal axis (101) of the rupturing tool (100), that is, to a neutral position; and lastly the rupturing tool (100) would be removed from the straight bone tunnel (4) with a first widening segment and a second widening segment (5, 6).

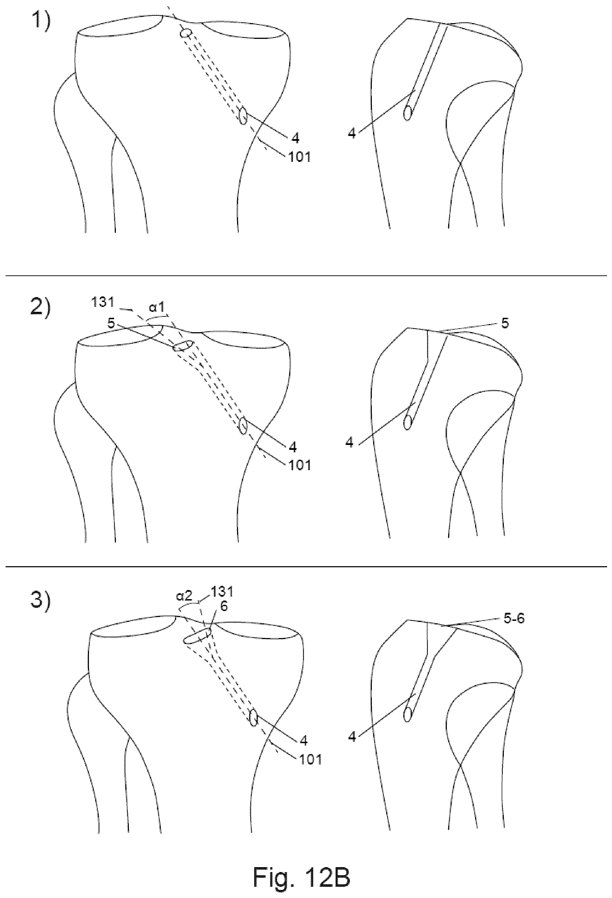

Figure 12b shows, in detail, the straight bone tunnel (4) with a first widening segment and a second widening segment (5, 6) carved in the tibia:
1) perspective views of the straight bone tunnel (4).
2) perspective views of the straight bone tunnel (4) with a first widening segment (5) carved by means of the rupturing tool (100) of the invention.
3) perspective views of the straight bone tunnel (4) with a first widening segment and a second widening segment (5, 6) carved by means of the rupturing tool (100) of the invention.

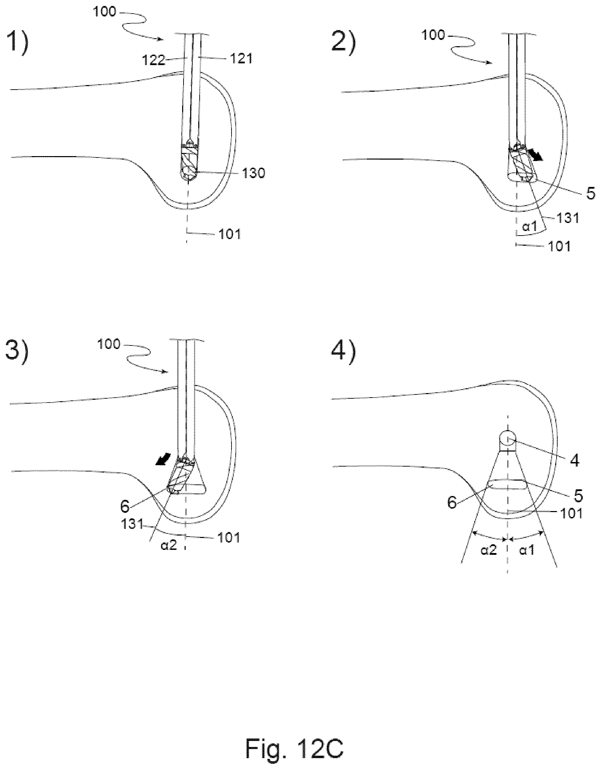

Figure 12c shows the steps of the preceding method performed on the femur:
1. point 1 shows the straight bone tunnel (4) with an inlet opening in the external bone cortex of the bone and an outlet opening in the internal bone cortex of said bone, created in the femur by means of the rupturing system (400, 500) of the invention. During this step, the axis of rupture (131) of the rupturing element (130) remains coaxial to the main longitudinal axis (101) of the rupturing tool (100);
2. point 2 shows how the rupturing element (130) is angled in a first direction with respect to the main longitudinal axis (101) of the rupturing tool (100), thereby carving a first widening segment (5) of the intraarticular outlet opening of the straight bone tunnel (4), defined by angle α1;
3. point 3 shows how the rupturing element (130) is angled in the direction opposite the first direction with respect to the main longitudinal axis (101) of the rupturing tool (100), thereby carving a second widening segment (6) of the intraarticular outlet opening of the straight bone tunnel (1), defined by angle α2;
4. point 4 shows a side view of the straight bone tunnel (4) carved in the femur, with a first widening segment and a second widening segment (5, 6).

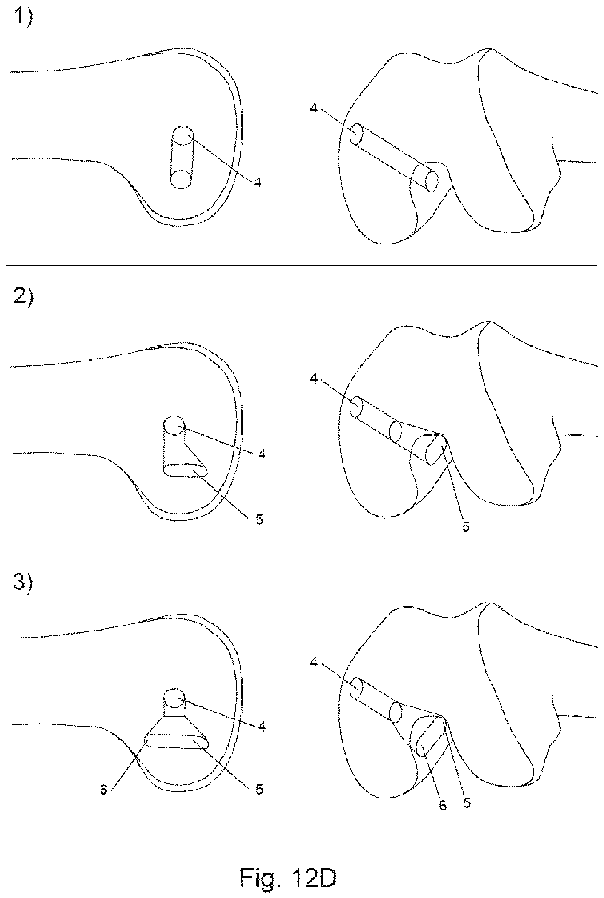

Figure 12d shows, in detail, the widening process:
1) perspective views of the straight bone tunnel (4);
2) perspective views of the straight bone tunnel (4) with a first widening segment (5) carved with the system (400, 500) of the invention; and
3) perspective views of the straight bone tunnel (4) with a first widening segment and a second widening segment (5, 6) carved with one of the systems (400, 500) of the invention.

In the particular example of reconstructing the anterior cruciate ligament, the intraarticular outlet opening of the bone tunnel to be created must be such that it allows the insertion of fibrous substance which, in the Caucasian population, generally has measurements of between 2 and 4 mm in thickness and between 12 mm and 18 mm in width. However, these particular measurement ranges provided in the preceding examples must be adapted based on the particular anatomy of other populations, based on the particular anatomy of specific patients, and based on the type of implant and/or technique used, and/or on the final application for which the rupturing tool is used, in medicine or veterinary science.

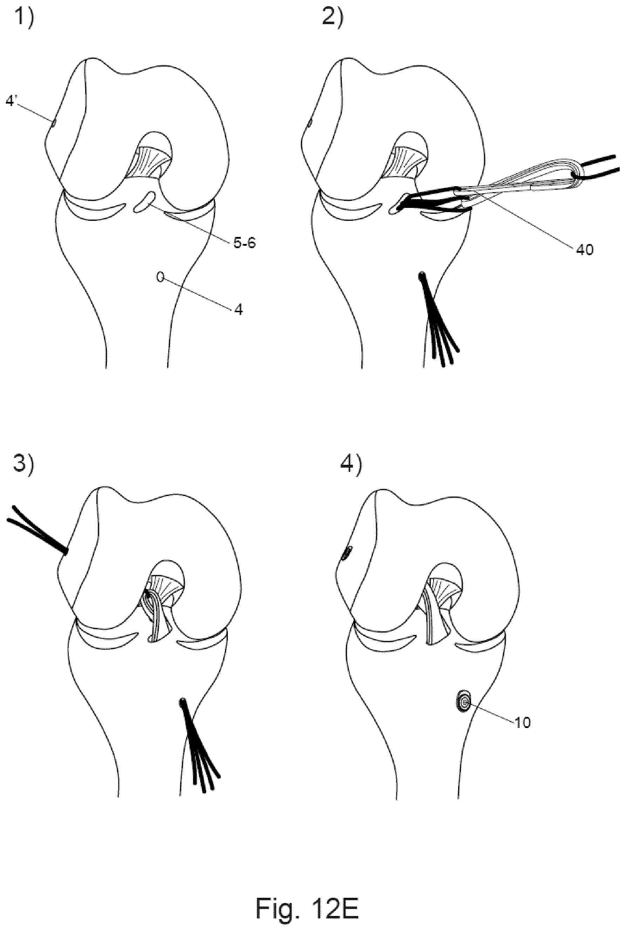

Figure 12e shows a general view of the steps of a method for restoring the anterior cruciate ligament (ACL) of the right knee. In particular, step 1) shows a general view of the joint with the bone tunnels anatomically widened by means of the rupturing tool (100) of the invention. Step 2) illustrates the introduction in the tibial bone tunnel of the sutures with which both branches of the quadruple-folded semitendinosus graft (500) are pulled into the tibial bone tunnel. Step 3) shows the sutures at the femoral end of the implant being introduced in the femoral bone tunnel. Step 4) illustrates the anatomical twisting of both branches of the implant.

In a particular embodiment, the fixing of the femoral end of the quadruple implant (40) is performed by means of a fixed-loop cortical button and the fixing of the sutures from which both tibial ends of the graft hang is performed by means of retaining the sutures corresponding to each of the branches of the graft to both sides of a cortical fixing device (10) such as the one described in European patent application EP3897455 A1.

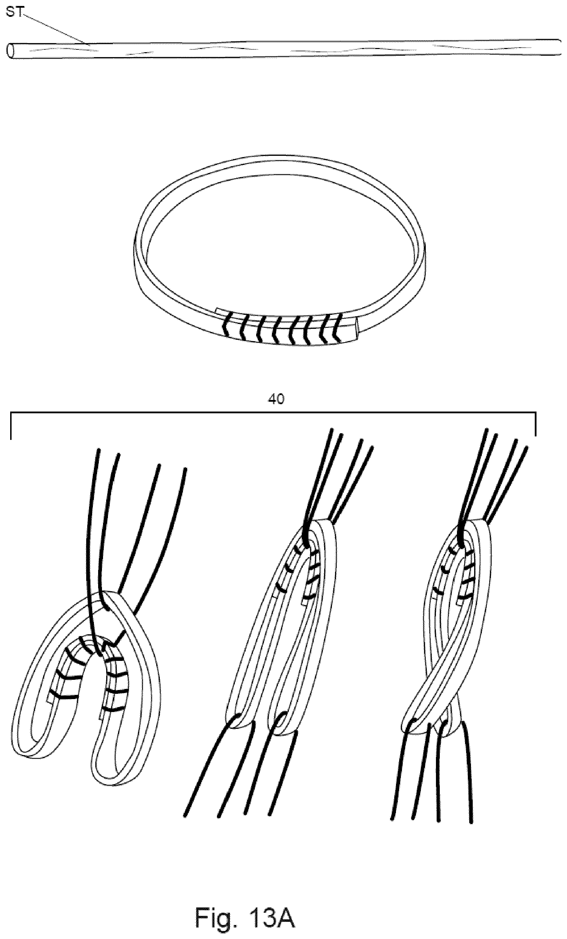

Figure 13a shows the configuration of a quadruple implant (40) from a semitendinosus graft (ST) for restoring an ACL.

In a particular embodiment, not shown in the figures, a small rigid cannula through which the suture is passed is used, such that the pulling force that the sutures exert on the folded ends of the implant is distributed homogenously, which facilitates the introduction of these folded ends in the widening segments of their respective bone tunnels created by means of the system (400, 500) of the invention.

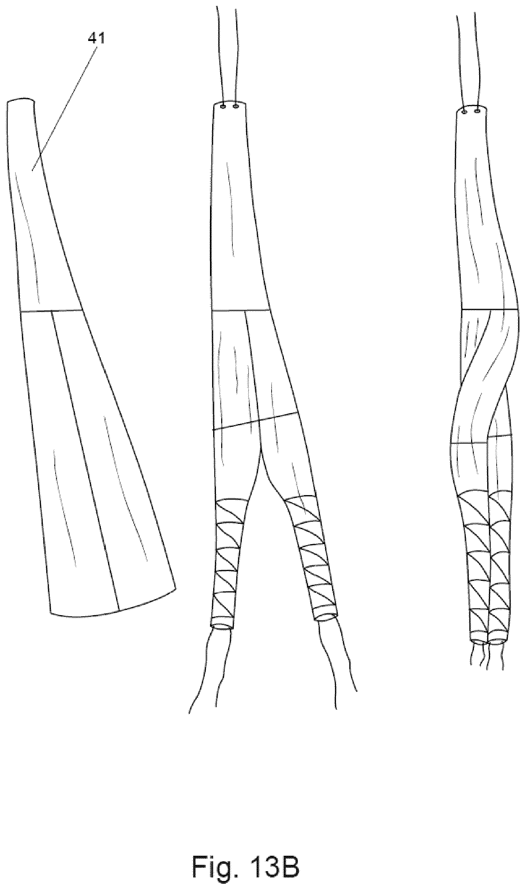

Figure 13b shows the configuration of an implant with two branches from an Achilles tendon allograft (41) for restoring an ACL.

Figure 13c shows the configuration of an implant with two branches from a quadriceps tendon graft (42) comprising a bone plug for restoring an ACL.

## Claims

1. Bilateral rupturing tool (100) for minimally invasive surgical interventions comprising:
• a longitudinal element (120) oriented according to a main longitudinal axis (101) which in turn comprises:
o a first longitudinal body (121) configured for performing a linear movement along the main longitudinal axis (101) and comprising a first distal end (121.2),
o a second longitudinal body (122), arranged parallel to the first longitudinal body (121), configured for performing a linear movement along the main longitudinal axis (101) and comprising a second distal end (122.2);
• a rupturing assembly which in turn comprises:
o a rupturing element (130) comprising an axis of rupture (131) and configured for receiving and performing a rupturing movement about the axis of rupture (131) and for performing an angular movement, forming a plurality of angles α with respect to the main longitudinal axis (101) comprised in a main plane (101'), said main plane (101') containing the main longitudinal axis (101) and the axis of rupture (131); and
o a movement transfer element (140), comprising a distal portion (140.1), wherein the movement transfer element (140):
is arranged inside the longitudinal element (120), between the first longitudinal body (121) and the second longitudinal body (122);
is attached to the rupturing element (130) by the distal portion (140.1), and
is configured for receiving and performing a rupturing movement and for transferring the rupturing movement to the rupturing element (130),
• an articulated attachment means (150) which comprises
a first section (150.1) attached to the first distal end (121.2) of the first longitudinal body (121);
a second section (150.2) attached to the second distal end (122.2) of the second longitudinal body (122); and
a central section (151) comprising a through hole (151.1) with the rupturing assembly going through same, the rupturing assembly being fixed to the inside of the through hole (151.1);
wherein the articulated attachment means (150) is configured for performing an angular movement in the main plane (101') of the rupturing tool (100), such that:
- when the first longitudinal body (121) performs linear movement in the proximal-distal direction, the second longitudinal body (122) performs linear movement in the distal-proximal direction and the rupturing element (130) performs angular movement in the main plane (101') in a first direction, and
- when the first longitudinal body (121) performs linear movement in the distal-proximal direction, the second longitudinal body (122) performs linear movement in the proximal-distal direction and the rupturing element (130) performs angular movement in the main plane (101') in the direction opposite the first direction.

2. Bilateral rupturing tool (100) according to the preceding claim, wherein the rupturing assembly goes through and is fixed to the inside of the through hole (151.1):
• by means of the rupturing element (130); or
• by means of the movement transfer element (140); or
• partially by means of the rupturing element (130) and partially by means of the movement transfer element (140); or
• by means of a bearing fitted in the through hole (151.1); or
• by means of a combination of two or more of the foregoing.

3. Bilateral rupturing tool (100) according to any of the preceding claims, wherein the movement transfer element (140) and/or the rupturing element (130) are cannulated.

4. Bilateral rupturing tool (100) according to any of the preceding claims, further comprising at least a first attachment and/or coupling means (160) configured for attaching and/or coupling the movement transfer element (140) to a rupturing movement generator device (200, 200').

5. Bilateral rupturing tool (100) according to any of the preceding claims, further comprising
• a support body (110) surrounding, at least partially, the longitudinal element (120), and
• a second attachment and/or coupling means (170) configured for attaching and/or coupling the support body (110) to a rupturing movement generator device (200, 200').

6. **Bilateral** rupturing tool (100) according to any of the preceding claims, further comprising a rupturing guide (300) configured for guiding the longitudinal element (120) from an original position to at least one target position, wherein the rupturing guide (300) comprises:
- a tubular guide (310) comprising:
o a longitudinal conduit (311) with a third proximal end (311.1) and a third distal end (311.2); the longitudinal conduit (311) comprising a distal appendage (311.3) at the third distal end (311.2); and
o a striking edge (312), a prolongation of the longitudinal conduit (311) at the third proximal end (311.1); and
wherein the longitudinal conduit (311) is configured for housing therein the longitudinal element (120), and
wherein the tubular guide (310) is oriented according to a first longitudinal guiding axis (301); and
the striking edge (323) is configured for receiving a striking force in the direction of the first longitudinal guiding axis (301); and
- a guide arch (320) comprising:
o a proximal arch portion (321) comprising a first coupling means (330) configured for securely coupling and uncoupling the proximal arch portion (321) with respect to the tubular guide (310), in a plurality of different positions along the tubular guide (310),
o a distal arch portion (322) in turn comprising a distal tip (322.1) and a distal point (322.2) through which the first longitudinal guiding axis (301) passes; and
o a second coupling means (340) configured for coupling and uncoupling the distal arch portion (322) with respect to the proximal arch portion (321) in a plurality of different positions, such that starting from a first position according to which the tubular guide (320) is oriented according to a first longitudinal guiding axis (301), when the second coupling means (340) is in a position other than the first position according to a second longitudinal guiding axis (301.1), it forms an angle β with the first longitudinal axis (301).

7. Bilateral rupturing tool (100) according to the preceding claim, wherein
the tubular guide (310) further comprises a first longitudinal hole (313) and a second longitudinal hole (313');
the first longitudinal body (121) further comprises a first projection (121.3); and
the second longitudinal body (122) further comprises a second projection (122.3);
wherein the first longitudinal hole (313) is sized and configured for receiving and guiding the first projection (121.3) from a proximal location to a distal location, and vice versa; and
the second longitudinal hole (313') is sized and configured for receiving and guiding the second projection (122.3) from a proximal location to a distal location, and vice versa.

8. The bilateral rupturing tool (100) according to any of claims 6 or 7, wherein the distal appendage (311.3) comprises:
• a trilobular tip; or
• two tips; or
• a beveled recess; or
• polyhedral faces.

9. The bilateral rupturing tool (100) according to any of the preceding claims, further comprising an actuator (180,190) configured for generating and transmitting a linear movement to the first longitudinal body (121) and to the second longitudinal body (122) such that both longitudinal bodies (121, 122) move linearly in opposite directions.

10. The bilateral rupturing tool (100) according to claim 9, wherein:
- the first longitudinal body (121) comprises a first longitudinal notch (121.4) configured for cooperating with the actuator (180); and
- the second longitudinal body (122) comprises a second longitudinal notch (122.4) configured for cooperating with the actuator (180);
and wherein the actuator (180) comprises:
- a first rod (181) configured for penetrating and cooperating with the first longitudinal notch (121.4);
- a second rod (182) configured forp enetrating and cooperating with the second longitudinal notch (122.4); and
- an actuation trigger (180.1) attached to the first rod (181);
wherein the first rod (181) and the second rod (182) are arranged in parallel and attached to one another by two longitudinal parts (183, 184) at the ends thereof such that:
- when the actuation trigger (180.1) performs a rotational movement in the proximal-distal direction with respect to an axis of rotation (185) located between the first rod (181) and the second rod (182),
the first rod (181) performs a rotational movement in the proximal-distal direction, causing the first longitudinal body (121) to perform a proximal-distal linear movement; and
the second rod (182) performs a rotational movement in the distal-proximal direction, causing the second longitudinal body (122) to perform a distal-proximal linear movement; and
- when the actuation trigger (180.1) performs a rotational movement in the distal-proximal direction with respect to the axis of rotation (185),
the first rod (181) performs a rotational movement in the distal-proximal direction, causing the first longitudinal body (121) to perform a distal-proximal linear movement; and
the second rod (182) performs a rotational movement in the proximal-distal direction, causing the second longitudinal body (122) to perform a proximal-distal linear movement.

11. Bilateral rupturing tool (100) according to claim 10, further comprising positioning and securing means for the actuator (180),
wherein the positioning and securing means for the actuator (180) are configured for positioning and securing the actuator (180) in a plurality of different positions, such that, for each of said positions, the distance between the first distal end (121.1) and the second distal end (122.1) of both longitudinal bodies (121, 122) is different.

12. Bilateral rupturing tool (100) according to any of claims 9 to 11, wherein the actuator (180, 190) further comprises a return spring configured for exerting a return force on the first longitudinal body and the second longitudinal body (121, 122) such that the relative movement between said bodies (121, 122) is substantially zero.

13. Bilateral rupturing tool (100) according to claim 9, wherein:
- the first longitudinal body (121) comprises a first outer thread (191) configured for cooperating with the actuator (190); and
- the second longitudinal body (122) comprises a second outer thread (192) configured for cooperating with the actuator (190); wherein the first outer thread (191) and the second outer thread (192) have opposite thread directions;
and wherein the actuator (190) comprises
- a nut (193) which in turn comprises therein:
o a first inner thread portion (193.1), matching the first outer thread (191); and
o a second inner thread portion (193.2), matching the second outer thread (192); wherein the first inner thread portion (193.1) and the second inner thread portion (193.1) have opposite thread directions;
wherein the nut (193) is configured for cooperating with the first longitudinal body (121) and the second longitudinal body (122) such that the assembly works like a double spindle mechanism;
- an actuation control (194) attached to the nut (193);
wherein
- when the actuation control (194) performs a rotational movement in the thread direction of the first inner thread portion (193.1), the first longitudinal body (121) performs a proximal-distal linear movement and the second longitudinal body (122) performs a distal-proximal linear movement; and
- when the actuation control (194) performs a rotational movement in the thread direction of the second inner thread portion (193.1), the first longitudinal body (121) performs a distal-proximal linear movement and the second longitudinal body (122) performs a proximal-distal linear movement.

14. Bilateral rupturing tool (100) according to any of the preceding claims, further comprising a suction line couplable to an external suction device for the removal of tissues.

15. Bilateral rupturing tool (100) according to any of the preceding claims, wherein the first longitudinal body (121) and/or the second longitudinal body (122) are rigid and/or straight.

16. Bilateral rupturing system (400) for minimally invasive surgical interventions, comprising:
- a rupturing movement generator device (200) configured for generating and performing a rupturing movement;
- at least one rupturing tool (100) according to any of the preceding claims, couplable to the rupturing movement generator device (200) at least by means of the movement transfer element (140);
and wherein the rupturing movement generator device (200) is further configured for transferring the rupturing movement to the movement transfer element (140).

17. Bilateral rupturing system (500) for minimally invasive surgical interventions comprising:
- a rupturing movement generator device (200') configured for generating and performing a rupturing movement;
- at least one rupturing tool (100) according to any of claims 5 to 15, couplable to the rupturing movement generator device (200') by means of the movement transfer element (140) and by means of the support body (110); wherein
the rupturing movement generator device (200') is configured for transferring the rupturing movement to the movement transfer element (140); and wherein
the rupturing movement generator device (200') further comprises an actuator (180', 190') configured for generating and transmitting a linear movement to the first longitudinal body (121) and to the second longitudinal body (122) such that both longitudinal bodies (121, 122) move linearly in opposite directions upon receiving the linear movement from the actuator (180', 190').
